# EUROPEAN PATENT SPECIFICATION

(11) **EP 0 327 239 B1**
(45) Date of publication and mention of the grant of the patent: **22.06.1994**
(21) Application number: 89300640.3
(22) Date of filing: 24.01.1989
(51) Int. Cl.: C07D 471/04, C07D 519/00, A61K 31/435

(54) **1-carbacephalosporin antibiotics**
1-Carbacephalosporin-Antibiotika
Antibiotiques dérivés de 1-carbacéphalosporine

(30) Priority: 25.01.1988 US 147471
(43) Date of publication of application: 09.08.1989
(73) Proprietor: ELI LILLY AND COMPANY, Indianapolis, Indiana 46285 (US)
(72) Inventor: Cook, Gwendolyn Kay, Indianapolis Indiana 46227 (US); McDonald, John Hampton, III, Indianapolis Indiana 46220 (US)
(74) Representative: Hudson, Christopher Mark

(56) References cited:
- US-A- 4 013 651
- CHEMICAL ABSTRACTS, vol. 106, no. 5, February 2, 1987, page 515, abstract-no. 32696e, Columbus, Ohio, US & JP-A-6197290.

## Description

This invention relates to 1-carba(dethia)cephalosporin antibiotics, intermediates for the preparation thereof, to pharmaceutical formulations comprising the antibiotics, and to a method for the treatment of infectious diseases in man and animals.

The 1-carba(dethia)cephalosporin antibiotics have the bicyclic ring system represented by the following formula wherein the numbering system is that commonly employed in the arbitrary cepham nomenclature system. 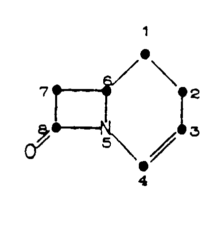 The 1-carba(1-dethia)cephalosporins are referred to herein for convenience as 1-carbacephalosporins or as 1-carba-3-cephem-4-carboxylic acids or numbered derivatives thereof. Alternatively, the compounds are 3,7- disubstituted derivatives of 2-carboxy-8-oxo-1-azabicyclo[4.2.0]oct-2-enes.

The preparation of 1-carbacephalosporins and C-3 substituted methyl derivatives thereof is taught broadly by Christensen et al., in U.S. Patent No. 4,226,866. Hirata et al., in U.K. patent application No. 2041923, teach a method for preparing 3-H and 3-halo 1-carbacephalosporins, while Hatanaka et al., Tetrahedron Letters, 24, No. 44, pp. 4837-4838 (1983), teach a method for preparing a 3-hydroxy-(±)-1-carbacephalosporin. A variety of 3-hydroxy-1-carbacephalosporins are also provided in EPO Patent Application Publication 209,352 while their 3-triflate (3-trifluoromethanesulfonic acid) esters are disclosed in EPO Patent Application Publication 211,540.

Certain cephalosporin compounds substituted at the 3-position with a quarternary ammonium methyl group are disclosed in Chemical Abstracts, vol. 106, no. 5, February 2, 1987, page 515, abstract 32696e and JP-A-61 97 290.

Although many safe and potent antibiotics of the ,8-lactam class are known and used clinically, the research into this class continues in an effort to find antibiotics with improved efficacy, particularly against microorganisms insensitive or resistant to the known antibiotics.

This invention provides 7β-acylamino-1-carba-3-cephem-4-carboxylic acids substituted in the 3-position by a quaternary ammonium group, and esters, solvates, and salts thereof, useful as broad spectrum antimicrobial compounds. The invention comprises formulations of the 1-carbacephalosporins useful in a therapeutic method for the treatment of infectious diseases of man and animals caused by gram-positive and gram-negative bacteria.

The 1-carbacephalosporins provided by this invention are represented by the Formula I 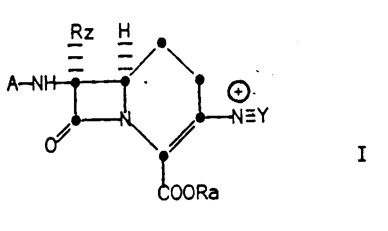 wherein A is hydrogen, an amino-protecting group, or an acyl group 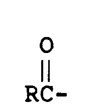 wherein R is hydrogen, C₁-C₆ alkyl, C₁-C₆ alkyl substituted by cyano, carboxy, halogen, amino, C₁-C₄ alkoxy, C1-C4 alkylthio, trifluoromethyl, or trifluoromethylthio, naphthyl, an optionally substituted phenyl group represented by the formula 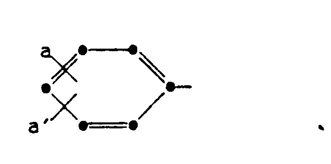 wherein a and a' independently are hydrogen, halogen, hydroxy, C₁-C₄ alkoxy, C₁-C₄ alkanoyloxy, C₁-C₄ alkyl, C1-C4 alkylthio, amino, C₁-C₄ alkanoylamino, C₁-C₄ alkylsulfonylamino, carboxy, carbamoyl, hydroxymethyl, aminomethyl, or carboxymethyl; a group represented by the formula 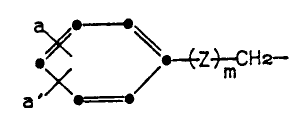 wherein a and a' have the same meanings as defined above, Z is O or S, and m is 0 or 1; a heteroarylmethyl group represented by the formula
Ri-CH₂-
wherein R₁ is thienyl, furyl, benzothienyl, benzofuryl, indolyl, triazolyl, tetrazolyl, oxazolyl, thiazolyl, oxadiazolyl, thiadiazolyl, and such heteroaryl groups substituted by amino, hydroxy, halogen, C₁-C₄ alkyl, C₁-C₄ alkoxy, C₁-C₄ alkylsulfonylamino; a substituted methyl group represented by the formula 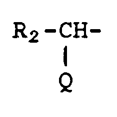 wherein R₂ is cyclohex-1,4-dienyl, or an optionally substituted phenyl group represented by the formula 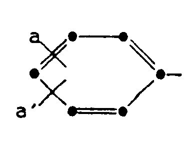 wherein a and a' have the above defined meanings, or R₂ is R₁ as defined above, and Q is hydroxy, C₁-C₄ alkanoyloxy, carboxy, sulfo, amino, or a substituted amino group represented by the formula 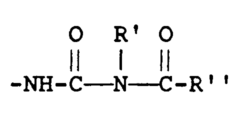 wherein R" is C₁-C₄ alkyl, furyl, thienyl, phenyl, halophenyl, nitrophenyl, styryl, halostyryl, nitrostyryl or a group 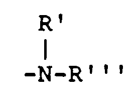 wherein each R' is independently hydrogen or C₁-C₃ alkyl, and R is hydrogen, C₁-C₃ alkylsulfonyl, C₁-C₃ alkyl, or C₁-C₄ alkanoyl; or Q is a substituted amino group represented by the formula 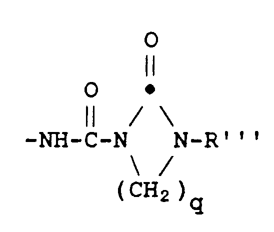 wherein R has the same meaning as defined above and q is 2 or 3; or Q is a substituted amino group represented by the formula 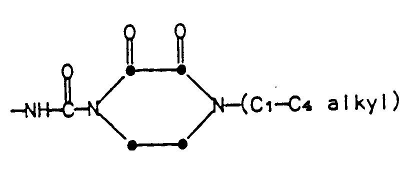 or a benzamido group represented by the formula 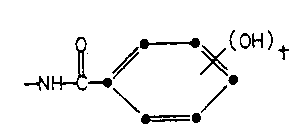 wherein t is 1 to 3; a pyridone group 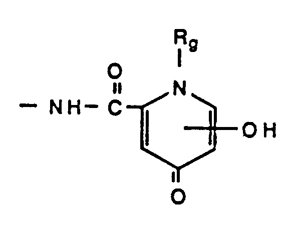 a pyridyl group 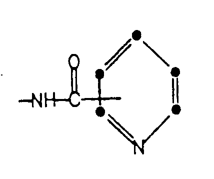 or said pyridyl group substituted by C₁-C₄ alkyl, amino, carboxy, hydroxy or halogen; an imidazolyl or pyrazolyl group 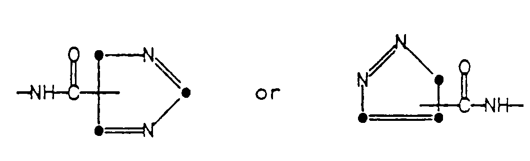 or said imidazolyl or pyrazolyl substituted by C₁-C₄ alkyl, carboxy, amino, or halogen; a benzpyridazin-4-one group or tautomer thereof 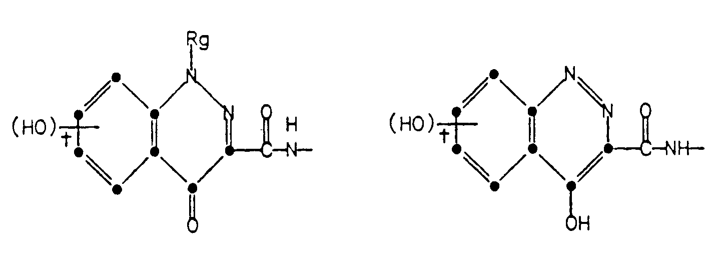 wherein Rg is hydrogen or C₁-C₃ alkyl, and t is 1 to 3; or a benzpyranone group 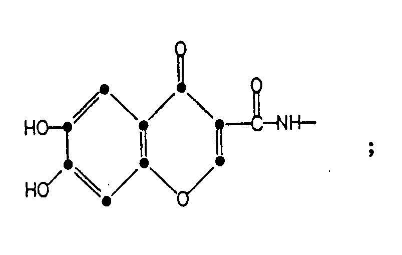 or R is a keto group or an oximino-substituted group represented by the formulae 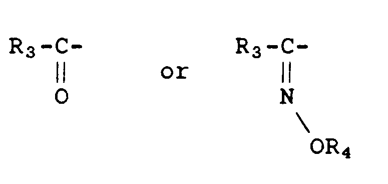 wherein R₃ is R₁ or R₂ as defined above and R₄ is hydrogen, C₁-C₄ alkyl, or a carboxy-substituted alkyl or cycloalkyl group represented by the formula 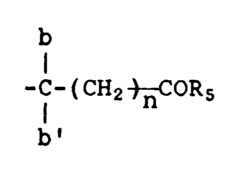 wherein b and b' independently are hydrogen or C₁-C₃ alkyl, or b and b', when taken together with the carbon to which they are bonded, form a 3- to 6-membered carbocyclic ring, n is 0-3, and R₅ is hydroxy, C₁-C₄ alkoxy, amino, C₁-C₄ alkylamino, or di(C₁-C₄ alkyl)amino; Rₐ is a negative charge, hydrogen, a biologically labile group, or a carboxy-protecting group; R_{z} is hydrogen, C₁-C₄ alkoxy, C₁-C₄ alkylthio, or a formamido group, -NHCHO;
- 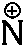 ≡Y is a quaternary ammonium group which is
   a) a pyridinium ring, which may be substituted with one or two of the following substituents: C₁ to C₆ alkyl, C₁ to C₆ substituted alkyl, C₃ to C₇ cycloalkyl, C₃ to C₇ substituted cycloalkyl, C₅ to C₇ cycloalkenyl, C₅ to C₇ substituted cycloalkenyl, halo, cyano, hydroxy, protected hydroxy, C₁ to C₄ alkoxy, C₁ to C₄ alkythio, C₁ to C₄ alkylsulfoxide, C₁ to C₄ alkylsulfonyl, phenyl, substituted phenyl, formyl, C₂ to C₄ alkanoyl, benzyl, benzoyl, amino, protected amino, C₁ to C₄ alkylamino, di(Cᵢ to C₄ alkyl)amino, trifluoromethyl, carboxy, protected carboxy, C₁ to C₄ alkoxycarbonyl, aminomethyl, protected aminomethyl, carboxymethyl, protected carboxymethyl, carbamoyl, which may be substituted once or twice with a C₁ to C₆ alkyl group, an aminosulfonyl group (which may be substituted once or twice with a C₁ to C₆ alkyl group), a sulfonic acid, or a substituted or unsubstituted cyclic C₂ to C₁₀ alkylene or heteroalkylene group;
   b) a quinolinium, isoquinolinium, (1 or 2)-pyradizinium, (1 or 3)-pyrimidinium, pyrazinium, N³-(C₁-C₄ allyl)imidazolinium, pyridazinium, phthalazinium, quinazolinium, purinium, thiazolinium, isothiazolinium, oxazolinium, isoxazolinium, (3 or 4)-1,3,4-thiadiazolinium, (2 or 4)-1,2,4-thiadiazolinium, (2 or 5)-1,2,5-thiadiazolinium, (3 or 4)-1,3,4-oxadiazolinium, (2 or 4)-1,2,4-oxadiazolinium, or a (2 or 5)-1,2,5-oxadiazolinium ring, or the mono or di-substituted derivatives thereof, wherein the substituents can be the same or different (and in the case of the quinolinium or isoquinolinium rings, on one or both rings) and are amino, protected amino, C₁ to C₄ alkylamino, di(Cᵢ to C₄ alkyl)amino, hydroxy, protected hydroxy, C₁ to C₄ alkoxy, C₁ to C₄ alkylthio, C₁ to C₄ alkylsulfoxide, C₁ to C₄ alkylsulfonyl, cyano, halo, C₁ to C₆ alkyl, C₁ to C₆ substituted alkyl, trifluoromethyl, C₃ to C₇ cycloalkyl, C₃ to C₇ substituted cycloalkyl, C₅ to C₇ cycloalkenyl, C₅ to C₇ substituted cycloalkenyl, sulfonic acid, carboxy, protected carboxy, C₁ to C₄ alkoxycarbonyl, hydroxy-(C₁ to C₃ alkyl), protected hydroxy-(C₁ to C₃ alkyl), formyl, C₂ to C₄ alkanoyl, an aminosulfonyl group (which may be substituted once or twice with a C₁ to C₆ alkyl group), carbamoyl (which may be substituted once or twice with a C₁ to C₆ alkyl group), aminomethyl, protected aminomethyl, carboxymethyl, (protected carboxy)methyl, phenyl, substituted phenyl, benzoyl or benzyl;
   c) a group of the formula 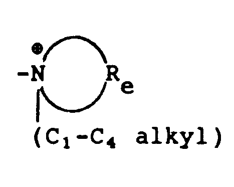 wherein Rₑ together with the nitrogen atom to which it is attached form a saturated or partially unsaturated 4 to 10 membered heterocyclic ring which may contain one or more further heteroatoms selected from oxygen, nitrogen or sulfur and wherein the substituent may be C₁ to C₆ alkyl, C₁ to C₆ substituted alkyl, C₃ to C₇ cycloalkyl, C₃ to C₇ substituted cycloalkyl, C₅ to C₇ cycloalkenyl, C₅ to C₇ substituted cycloalkenyl, halo, cyano, hydroxy, protected hydroxy, C₁ to C₄ alkoxy, C₁ to C₄ alkylthio, C₁ to C₄ alkylsulfoxide, C₁ to C₄ alkylsulfonyl, phenyl, substituted phenyl, formyl, C₂ to C₄ alkanoyl, benzyl, benzoyl, amino, protected amino, C₁ to C₄ alkylamino, di(C₁ to C₄ alkyl)amino, trifluoromethyl, carboxy, protected carboxy, C₁ to C₄alkoxycarbonyl, aminomethyl, protected aminomethyl, carboxymethyl, protected carboxymethyl, carbamoyl (which may be substituted once or twice with a C₁ to C₆ alkyl group), aminosulfonyl (which may be substituted once or twice with a C₁ to C₆ alkyl group), or sulfonic acid, or the benzo-fused analogs of the substituted or unsubstituted, saturated or partially unsaturated ring, or
   d) - 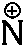 ≡Y is tri(C₁-C₄ alkyl) ammonium;
      or a solvate or a pharmaceutically acceptable non-toxic salt thereof.

This invention also provides a process for preparing a compound of the Formula I as defined above, or a solvate or a pharmaceutically acceptable non-toxic salt thereof, which comprises
A) reacting a 3-triflate ester of the formula 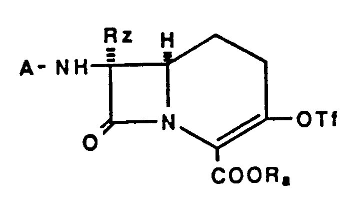 wherein Tf is a triflate moiety (-S0₂CF₃) and A and Rₐ are other than hydrogen with an amine N=Y and, optionally, deprotecting the resulting compound; or
B) esterifying a compound of the formula 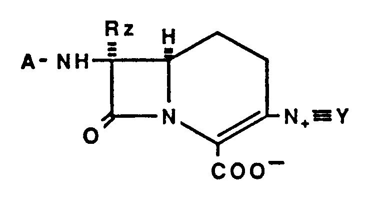 wherein A is other than hydrogen with a group Rₐ⁺; or
C) reacting a compound of the formula 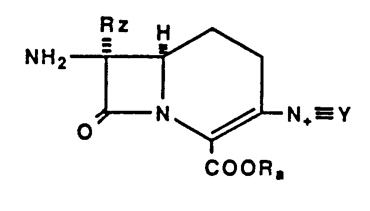 with a reagent AX wherein A is other than hydrogen and X is a good leaving group.

The compounds represented by the formula I wherein A is an acyl group, RCO, and Rₐ is hydrogen, a negative charge, or a biologically labile group and the pharmaceutically acceptable salts thereof, inhibit the growth of microorganisms pathogenic to man and animals. The compounds which are protected (A = an amino protecting group), deprotected (A = hydrogen), or are in esterified form (Rₐ = carboxy-protecting group) are useful as intermediates as described hereinafter.

In the above definition of the compounds represented by the formula I, "Cᵢ-C₆ alkyl" refers to the straight and branched chain alkyl groups such as methyl, ethyl, n-propyl, isopropyl, n-butyl, t-butyl, n-pentyl, n-hexyl, 3-methylpentyl, and like alkyl groups; "C₁-C₆ alkyl substituted by cyano" refers to cyanomethyl, cyanoethyl, 4-cyanobutyl, and the like; "Cᵢ -C₆ alkyl substituted by ... carboxy" refers to such groups as carboxymethyl, 2-carboxyethyl, 2-carboxypropyl, 4-carboxybutyl, 5-carboxypentyl, and the like; "C₁-C₆ alkyl substituted by ... halogen" refers to chloromethyl, bromomethyl, 2-chloroethyl, 1-bromoethyl, 4-chlorobutyl, 4-bromopentyl, 6-chlorohexyl, 4-fluorobutyl, 3-fluoropropyl, fluoromethyl, and the like; "Cᵢ-C₆ alkyl substituted by ... amino" refers to such groups as 2-aminoethyl, aminomethyl, 3-aminopropyl and 4-aminobutyl; "Cᵢ -C₆ alkyl substituted by ... C₁-C₄ alkoxy" refers to methoxymethyl, 2-methoxyethyl, 2- ethoxyethyl, ethoxymethyl, 3-propoxypropyl, 3-ethoxy-butyl, 4-t-butyloxybutyl, 3-methoxypentyl, 6-methoxyhexyl, and like group; "Cᵢ -C₆ alkyl substituted by ... C, -C4-alkylthio" refers to such groups as for example methylthiomethyl, 2-methylthioethyl, 2-ethylthiopropyl, 4-methylthiobutyl, 5-ethylthiohexyl, 3-t-butylthiopropyl, and like groups; "C1-C6 alkyl substituted by ... trifluoromethyl" is exemplified by 2,2,2-trifluoroethyl, 3,3,3-trifluoropropyl, 4,4,4-trifluorobutyl, and the like; and "Ci-Ce alkyl substituted by ... trifluoromethylthio" refers to, for example, trifluoromethylthiomethyl, 2-trifluoromethylthioethyl, 2-trifluoromethylthiopropyl, 4-trifluoromethylthiobutyl, 5-trifluoromethylthiohexyl, and like C₁-C₆ alkyl substituted groups.

The terms "halo" and "halogen" refer to the fluoro, chloro, bromo or iodo groups.

When, in the formula I, R is a substituted phenyl group wherein the substituent(s) are represented by a and a', examples of such groups are halophenyl such as 4-chlorophenyl, 3-bromophenyl, 2-fluorophenyl, 2,4-dichlorophenyl, and 3,5-dichlorophenyl; hydroxyphenyl such as 2-hydroxyphenyl, 3-hydroxyphenyl, 4-hydroxyphenyl, 2,4-dihydroxyphenyl, and 3,4-dihydroxyphenyl; alkoxyphenyl, such as 2,6-dimethoxyphenyl, 4-methoxyphenyl, 3-ethoxyphenyl, 3,4-dimethoxyphenyl, 4-t-butyloxyphenyl, 4-methoxy-3-ethoxyphenyl, and 4-n-propoxyphenyl; alkanoyloxyphenyl such as 2-acetoxyphenyl, 4-propionoxyphenyl, 4-formyloxyphenyl, 4-acetoxyphenyl, 3-butyryloxyphenyl, and 3-acetoxyphenyl; alkylphenyl such as 4-methylphenyl, 2-methylphenyl, 2,4-dimethylphenyl, 3-t-butylphenyl, 4-ethylphenyl, 4-ethyl-3-methylphenyl, and 3,5-dimethylphenyl; alkylthiophenyl such as 4-methylthiophenyl, 3-n-butylthiophenyl, 2-ethylthiophenyl, 3,4-dimethylthiophenyl, and 3-n-propylthiophenyl; aminophenyl such as 2-aminophenyl, 4-amino-phenyl, 3,5-diaminophenyl, and 3-aminophenyl; alkanoylaminophenyl such as 2-acetylaminophenyl, 4-acetylaminophenyl, 3-propionylaminophenyl, and 4-butyrylaminophenyl; alkylsulfonylaminophenyl such a 3-methylsulfonylaminophenyl, 4-methylsulfonylaminophenyl, 3,5-(dimethylsulfonylamino)phenyl, 4-n-butylsulfonylaminophenyl, and 3-ethylsulfonylaminophenyl; carboxyphenyl such as 2-, 3-, or 4-carboxyphenyl, 3,4-dicarboxyphenyl, and 2,4-dicarboxyphenyl; carbamoylphenyl such as 2-carbamoylphenyl, 2,4-dicarbamoylphenyl, and 4-carbamoylphenyl; hydroxymethylphenyl such as 4-hydroxymethylphenyl and 2-hydroxymethylphenyl; aminomethylphenyl such as 2-aminomethylphenyl and 3-aminomethylphenyl; and carboxyphenyl such as 2-carboxymethylphenyl, 4-carboxymethylphenyl, and 3,4-dicarboxymethylphenyl; and the substituted phenyl groups bearing different substituents such as 4-chloro-3-methylphenyl, 4-fluoro-3-hydroxyphenyl, 3,5-dichloro-4-hydroxyphenyl, 4-hydroxy-3-chlorophenyl, 4-hydroxy-3-methylphenyl, 4-ethyl-3-hydroxyphenyl, 4-methoxy-3-hydroxyphenyl, 4-t-butyloxy-2-hydroxyphenyl, 4-acetylamino-3-methoxyphenyl, 3-amino-4-ethylphenyl, 2-aminomethyl-4-chlorophenyl, 2-hydroxymethyl-3-methoxyphenyl, 2-hydroxymethyl4-fluorophenyl, 2-acetoxy-4-aminophenyl, 4-acetoxy-3-methoxyphenyl, 3-isopropylthio-4-chlorophenyl, 2-methylthio-4-hydroxymethylphenyl, 4-carboxy-3-hydroxyphenyl, 4-ethoxy-3-hydroxyphenyl, 4-methylsulfonylamino-2-carboxyphenyl, 4-amino-3-chlorophenyl, and 2-carboxymethyl-4-hydroxyphenyl.

Examples of RCO- groups of the formula I wherein R is a group represented by the formula 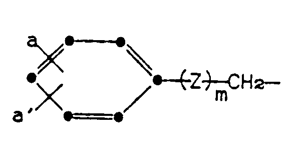 with m = 0 are: phenylacetyl, 4-hydroxyphenylacetyl, 4-chlorophenylacetyl, 3,4-dichlorophenylacetyl, 4-methoxyphenylacetyl, 3-ethoxyphenylacetyl, 2-aminomethylphenylacetyl, 3-carboxyphenylacetyl, 4-acetoxyphenylacetyl, 3-aminophenylacetyl, and 4-acetylaminophenylacetyl; and with m = 1 and Z = O, phenoxyacetyl, 4-chlorophenoxyacetyl, 4-fluorophenoxyacetyl, 3-aminophenoxyacetyl, 3-hydroxyphenoxyacetyl, 2-methoxyphenoxyacetyl, 2-methylthiophenoxyacetyl, 4-acetylaminophenoxyacetyl, 3,4-dimethylphenoxyacetyl, and 3-hydroxymethylphenoxyacetyl; and with m = 1 and Z = S, phenylthioacetyl, 4-chlorophenyl- thioacetyl, 3,4-dichlorophenylthioacetyl, 2-fluorophenylthioacetyl, 3-hydroxyphenylthioacetyl, and 4-ethox- yphenylthioacetyl.

Examples of Rᵢ-CH₂CO- groups of the formula I wherein R₁ is a heteroaryl group are: 2-thienylacetyl, 3-thienylacetyl, 2-furylacetyl, 2-benzothienylacetyl, 2-benzofurylacetyl, indol-2-ylacetyl, 1 H-tetrazol-1- ylacetyl, oxazol-2-ylacetyl, oxazol-4-ylacetyl, thiazol-4-ylacetyl, 2-aminothiazol-4-ylacetyl, 1,3,4-oxadiazol-2- ylacetyl, 1,3,4-thiadiazol-2-ylacetyl, 5-ethyl-1,3,4-thiadiazol-2-ylacetyl, and like heteroaryl groups optionally substituted by amino, C₁-C₄ alkylsulfonylamino, hydroxy, halo, C₁-C₄ alkyl or C₁-C₄ alkoxy groups.

Examples of RCO- groups of the formula I compounds wherein R is a substituted methyl group represented by the formula R₂-CH(Q)- and Q is amino, carboxy, hydroxy, or sulfo, are 2-carboxy-2-phenylacetyl, 2-amino-2-(2-naphthalenyl)acetyl, 2-carboxy-2-(4-hydroxyphenyl)acetyl, 2-amino-2-phenylacetyl, 2-amino-2-(4-hydroxyphenyl)acetyl, 2-amino-2-(3-chloro-4-hydroxyphenyl)acetyl, 2-amino-2-(cyclohex-1,4-dien-1-yl)acetyl, 2-hydroxy-2-phenylacetyl, 2-formyloxy-2-phenylacetyl, 2-sulfo-2-phenylacetyl, 2-sulfo-2-(4-methylphenyl)acetyl, and 2-acetoxy-2-(3-hydroxyphenyl)acetyl, 2-amino-2-(2-thienyl)acetyl, 2-amino-2-(3-benzothienyl)acetyl, 2-amino-2-(1 H-tetrazol-1-yl)acetyl, 2-hydroxy-2-(1,3,4-thiadiazol-2-yl)acetyl, 2-amino-2-(2-aminothiazol-4-yl)acetyl, 2-carboxy-2-(2-thienyl)acetyl, 2-carboxy-2-(benzothien-2-yl)acetyl, and 2-hydroxy-2-(benzofur-2-yl)acetyl; and when Q is a substituted amino group represented by the formula 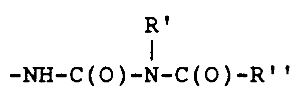 examples of such acyl groups are 2-(N-methyl-N-benzoylcarbamoylamino)-2-phenylacetyl, 2-(N-methyl-N-cinnamoylcarbamoylamino)-2-(2-furyl)acetyl, 2-(N,N-dimethylcarbamoylureido)-2-(4-chlorophenyl)acetyl, 2-[N-methyl-N-(2-chlorocinnamoyl)carbamoylamino]-2-(2-thienyl)acetyl, and 2-(N-ethyl-N-acetylcar- bamoylamino)-2-(4-hydroxyphenyl)acetyl; and when Q is a substituted amino group represented by the formula 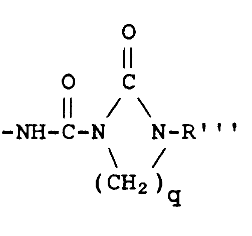 examples of acyl group RCO- are 2-[(3-methylimidazolidin-2-one-1-yl)carbonylamino]-2-phenylacetyl, 2-[(3- acetylimidazolidin-2-one-1-yl)carbonylamino]-2-phenylacetyl, 2-[(3-methylsulfonylimidazolidin-2-one-1-yl)-2-2-thienyl)acetyl, and 2-[(3-acetylhexahydropyrimidin-2-one-1-yl)carbonylamino]-2-phenylacetyl; and when Q is a hydroxy-substituted benzamido group represented by the formula 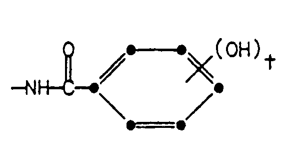 examples of such acyl groups are 2-(2,4-dihydroxybenzamido)-2-phenylacetyl, 2-(4-hydroxybenzamido)-2-(4-hydroxyphenyl)acetyl, 2-(3,4-dihydroxybenzamido)-2-(2-aminothiazol-4-yl)acetyl, 2-(3,5-dihydroxyben- zamido)-2-(3-thienyl)acetyl, and 2-(2-hydroxybenzamido)-2-(2-benzofuryl)acetyl.

Examples of RCO acyl groups of the compounds represented by formula I when R is a keto group or an oximino-substituted group represented by the formulae 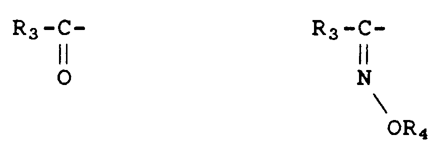 are the keto groups 2-oxo-2-phenylacetyl, 2-oxo-2-(2-thienyl)acetyl, 2-oxo-2-(2-aminothiazol-4-yl)acetyl; and oximino-substituted groups 2-phenyl-2-methoxyiminoacetyl, 2-(2-thienyl)-2-ethoxyiminoacetyl, 2-(2-furyl)-2-methoxyiminoacetyl, 2-(2-benzothienyl)-2-carboxymethoxyiminoacetyl, 2-(2-thienyl)-2-(2-carboxyethoxy)-iminoacetyl, 2-(2-amino-1,2,4-thiadiazol-4-yl)-2-methoxyiminoacetyl, 2-(2-aminothiazol-4-yl)-2-methoxyiminoacetyl, 2-(2-chlorothiazol-4-yl)-2-methoxyiminoacetyl, 2-(2-aminothiazol-4-yl)-2-(2-carboxyprop-2-yl)-oxyiminoacetyl, 2-(2-aminothiazol-4-yl)-2-(2-carbamoylprop-2-yl)oxyiminoacetyl, and 2-(5-amino-1,3,4-thiadiazol-2-yl)-2-methoxyiminoacetyl.

The term "carboxy-protecting group" as used in this specification refers to one of the ester derivatives of the carboxylic acid group commonly employed to block or protect the carboxylic acid group while reactions are carried out on other functional groups on the compound. Examples of such carboxylic acid protecting groups include benzyl, 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxybenzhydryl, 2,2',4,4'-tetramethoxybenzhydryl, t-butyl, t-amyl, trityl, 4-methoxytrityl, 4,4'-dimethoxytrityl, 4,4',4"-trimethoxytrityl, 2-phenylprop-2-yl, trimethylsilyl, t-butyldimethylsilyl, phenacyl, 2,2,2-trichloroethyl, ;8-(trimethy!si!y!)ethy!, β-(di(n-butyl)methylsilyl)ethyl, p-toluenesulfonylethyl, 4-nitrobenzylsulfonylethyl, allyl, cinnamyl, 1-(trimethylsilylmethyl)prop-1-en-3-yl, and like moieties. The species of carboxy-protecting group employed is not critical so long as the derivatized carboxylic acid is stable to the conditions of subsequent reaction(s) on other positions of the molecule and can be removed at the appropriate point without disrupting the remainder of the molecule. In particular, it is important not to subject the carboxy-protected 1-carbacephalosporin molecule to strong nucleophilic bases or reductive conditions employing highly activated metal catalysts such as Raney nickel. (Such harsh removal conditions are also to be avoided when removing amino-protecting groups and hydroxy-protecting groups, discussed below.) Preferred carboxylic acid protecting groups are the benzhydryl and allyl groups. Carboxy-protecting groups similar to those used in the cephalosporin, penicillin and peptide arts can also be used to protect a carboxy group substituents of the compounds provided herein. Further examples of these groups are found in E. Haslam, "Protective Groups in Organic Chemistry", J.G.W. McOmie, Ed., Plenum Press, New York, N.Y., 1973, Chapter 5, and T.W. Greene, "Protective Groups in Organic Synthesis", John Wiley and Sons, New York, N.Y., 1981, Chapter 5. A related term is "protected carboxy", which refers to a carboxy group substituted with one of the above carboxy-protecting groups.

The term "amino-protecting group" as used in the specification refers to substituents of the amino group commonly employed to block or protect the amino functionality while reacting other functional groups on the compound. Examples of such amino-protecting groups include the formyl group, the trityl group, the phthalimido group, the trichloroacetyl group, the chloroacetyl, bromoacetyl and iodoacetyl groups, urethane- type blocking groups such as benzyloxycarbonyl, 4-phenylbenzyloxycarbonyl, 2-methylbenzyloxycarbonyl, 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyloxycarbonyl, 4-bromobenzyloxycarbonyl, 3-bromobenzyloxycarbonyl, 4-nitrobenzyloxycarbonyl, 4-cyanobenzyloxycarbonyl, 2-(4-xenyl)iso-propoxycarbonyl, 1,1-diphenyleth-1-yloxycarbonyl, 1,1-diphenylprop-1-yloxycarbonyl, 2-phenylprop-2-yloxycarbonyl, 2-(p-toluyl)-prop-2-yloxycarbonyl, cyclopentanyloxycarbonyl, 1-methylcyclopentanyloxycarbonyl, cyclohexanyloxycarbonyl, 1-methylcyclohexanyloxycarbonyl, 2-methylcyclohexanyloxycarbonyl, 2-(4-toluylsulfonyl)-ethoxycarbonyl, 2-(methylsulfonyl)ethoxycarbonyl, 2-(triphenylphosphino)ethoxycarbonyl, 9-fluorenylmethoxycarbonyl ("FMOC"), 2-(trimethylsilyl)ethoxycarbonyl, allyloxycarbonyl, 1-(trimethylsilylmethyl)prop-1-en-3-yl-oxycarbonyl, 5-benzisoxalylmethoxycarbonyl, 4-acetoxybenzyloxycarbonyl, 2,2,2-trichloroethoxycarbonyl, 2-ethynyl-2-propoxycarbonyl, cyclopropylmethoxycarbonyl, 4-(decyloxy)benzyloxycarbonyl, isobornyloxycarbonyl, 1-piperidyloxycarbonyl and the like; the benzoylmethylsulfonyl group, the 2-(nitro)phenylsulfenyl group, the diphenylphosphine oxide group and like amino-protecting groups. The species of amino-protecting group employed is not critical so long as the derivatized amino group is stable to the condition of subsequent reaction(s) on other positions of the molecule and can be removed at the appropriate point without disrupting the remainder of the molecule. Preferred amino-protecting groups are the 1,2-bis-(dimethylsilyl)ethylene (See, e.g., U.S. Patent No. 4,558,124), benzyloxycarbonyl, p-nitrobenzyloxycarbonyl, allyloxycarbonyl, t-butoxycarbonyl, and trityl groups. Similar amino-protecting groups used in the cephalosporin, penicillin and peptide art are also embraced by the above terms. Further examples of groups referred to by the above terms are described by J.W. Barton, "Protective Groups In Organic Chemistry", J.G.W. McOmie, Ed., Plenum Press, New York, N.Y., 1973, Chapter 2, and T.W. Greene, "Protective Groups in Organic Synthesis", John Wiley and Sons, New York, N.Y., 1981, Chapter 7. The related term "protected amino" defines an amino group substituted with an amino-protecting group discussed above.

The 1-carbacephalosporins provided herein can be esterified with a biologically labile group to form esters which form the free acid antibiotic form in vivo. When in the formula I Rₐ is a biologically labile ester, Rₐ is an acyloxymethyl group represented by the formula 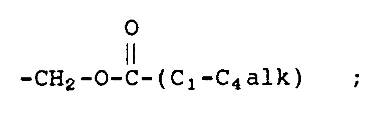 an acyloxyalkyl group represented by the formula 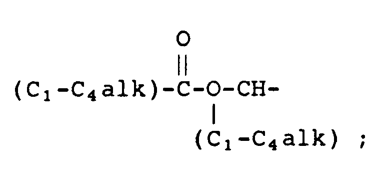 a dialkyl ether group represented by the formula (C₁-C₄alk)-O-CH₂CH₂-O-CH₂- ; phthalidyl, indanyl, or the 5-methyl-2-oxo-1,3-dioxolen4-methyl-4'-ylcyclocarbonate group represented by the formula

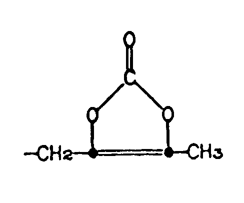

Examples of acyloxymethyl groups, Rₐ, are acetoxymethyl, propionoxymethyl and pivaloyloxymethyl. Acyloxyalkyl groups are exemplified by 1-acetoxyethyl, 1-acetoxypropyl, and 1-propionoxybutyl. Examples of dialkyl ether ester groups are β-methoxyethoxymethyl, β-ethoxyethoxymethyl, and β-t-butyloxyethox- ymethyl.

The biologically labile esters of the 1-carbacephalosporins can be used as pro-drugs and can provide ease of formulation and administration of the antibiotic.

Certain of the terms describing the substituents for the above preferred quaternary ammonium groups have already been defined. Specifically, the terms "Cᵢ to C₆ alkyl", "Cᵢ to C₆ substituted alkyl", "substituted phenyl", "halo", "Cᵢ to C₄ alkoxy", "protected carboxy", "protected hydroxy", and "protected amino" are as defined above for Formula I.

The substituent term "C₃ to C₇ cycloalkyl" includes the cyclopropyl, cyclobutyl, cyclopentyl, cyclohexyl or cycloheptyl rings. The substituent term "C₃ to C₇ substituted cycloalkyl" indicates the above cycloalkyl rings substituted by a halogen, hydroxy, protected hydroxy, C₁ to C₆ alkyl, C₁ to C₄ alkoxy, carboxy, protected carboxy, amino, or protected amino. The substituent term "C₅ to C₇ cycloalkenyl" indicates a 1, 2, or 3-cyclopentenyl ring, a 1, 2, 3 or 4-cyclohexenyl ring or a 1, 2, 3, 4 or 5-cycloheptenyl ring, while the term "substituted C₅ to C₇ cycloalkenyl" denotes the above C₅ to C₇ cycloalkenyl rings substituted by a C₁ to C₆ alkyl radical.

The substituent term "C₁ to C₄ alkylamino" refers to methylamino, ethylamino, n-propylamino, n-butylamino, iso-propylamino and the like. The substituent term "di(Cᵢ to C₄ alkyl)amino" denotes groups such as dimethylamino, diethylamino, methylethylamino, di(n-butyl)amino, di(n-propyl)amino and the like. Examples of the term "C₂ to C₄ alkanoyl group" are acetyl, n-propionyl, n-butyryl and the like. The substituent term "C₁ to C₄ alkoxycarbonyl" refers to groups such as methoxycarbonyl, ethoxycarbonyl, n-propoxycarbonyl, iso-propoxycarbonyl, n-butoxycarbonyl, t-butoxycarbonyl and the like.

The substituent term "C₁ to C₄ alkylthio" refers to sulfide groups such as methylthio, ethylthio, n-propylthio, iso-propylthio, n-butylthio, t-butylthio and like groups. The substituent term "Ci to C₄ alkylsulfoxide" indicates sulfoxide groups such as methylsulfoxide, ethylsulfoxide, n-propylsulfoxide, isopropylsulfoxide, n-butylsulfoxide, sec-butylsulfoxide, and the like.

The term "Cᵢ to C₄ alkylsulfonyl" encompasses groups such as methylsulfonyl, ethylsulfonyl, n-propylsulfonyl, iso-propylsulfonyl, n-butylsulfonyl, t-butylsulfonyl, and the like.

The term "hydroxy (C₁ to C₃ alkyl)" refers to C₁ to C₃ alkyl groups substituted at any position by a hydroxy group, such as hydroxymethyl, 2-hydroxyethyl, 1-hydroxyethyl, 3-hydroxy(n-propyl), 2-hydroxy(n-propyl), 1-hydroxy(n-propyl), 1-hydroxy(iso-propyl) and the like. Similarly, the term "protected hydroxy-(Ci to C₃ alkyl)" refers to C₁ to C₃ alkyl groups substituted at any position by a protected hydroxy group. Examples of such groups are exemplified when, in the above hydroxy (Cᵢ to C₃ alkyl groups), the term "hydroxy" is read as "protected hydroxy".

The substituent term "substituted or unsubstituted cyclic C₂ to C₁₀ alkylene or heteroalkylene group" defines such a cyclic group bonded ("fused") to the b or c face of the pyridinium ring. The cyclic group may be saturated or contain one or two double bonds. Furthermore, the cyclic group may have one or two alkylene groups replaced by one or two oxygen, nitrogen or sulfur atoms.

The cyclic alkylene or heteroalkylene group may be substituted once or twice by substituents selected from the group consisting of the following moieties: hydroxy, protected hydroxy, carboxy, protected carboxy, C₁ to C₄ alkoxycarbonyl, formyl, C₂ to C₄ alkanoyl, C₁ to C₆ alkyl, carbamoyl, C₁ to C₄ alkoxy, C₁ to C₄ alkylthio, C₁ to C₄ alkylsulfoxide, C₁ to C₄ alkylsulfonyl, halo, amino, protected amino, hydroxymethyl or a protected hydroxymethyl.

The cyclic alkylene or heteroalkylene group fused onto the pyridinium radical can contain two to ten ring members, but it preferably contains three to five members. Examples of such saturated cyclic groups are when the pyridinium group is fused to a cyclopentano, cyclohexano or cycloheptano ring. When the cyclic groups are unsaturated, examples occur when the pyridinium ring is fused to a cyclopenteno, cyclohexeno or cyclohepteno ring. Examples of fused cyclic groups which each contain one oxygen atom and one or two double bonds are when the pyridinium ring is fused to a furo, pyrano, dihydrofuro or dihydropyrano ring, and examples of cyclic groups which each have one sulfur atom and contain one or two double bonds are when the pyridinium ring is fused to a thieno, thiopyrano, dihydrothieno or dihydrothiopyrano ring. Examples of cyclic groups which contain two heteroatoms selected from sulfur and nitrogen and one or two double bonds are a pyridinium ring fused to a thiazolo, isothiazolo, dihydrothiazolo or dihydroisothiazolo ring. Examples of cyclic groups which contain two heteroatoms selected from oxygen and nitrogen and one or two double bonds are when the pyridinium ring is fused to an oxazolo, isoxazolo, dihydrooxazolo or dihydroisoxazolo ring. Examples of cyclic groups which contain two nitrogen heteroatoms and one or two double bonds occur when the pyridinium ring is fused to a pyrazolo, imidazolo, dihydropyrazolo or dihydroimidazolo ring.

The b or the c side of the pyridinium group can be fused to a cyclic group with three ring members. In the case of such a cyclic group containing only one heteroatom, the position of the heteroatom can result in a [2,3], [3,2] or [3,4] fusion with the pyridinium group. When the three-membered cyclic group contains two heteroatoms, the position of the heteroatoms can be such that they result in a [4,5], [5,4], [3,4] or [4,3] fusion with the pyridinium group.

Similarly, the b or c side of the pyridinium group can be fused to a cyclic group with four ring members. Such a cyclic group containing only one heteroatom can result in a [3,2], [2,3], [3,4] or [4,3] fusion with the pyridinium group. The four membered cyclic group with two heteroatoms can result in a [4,5], [5,4], [3,4], [4,3], [5,6] or [6,5] fusion to the pyridinium group.

Examples of the bicyclic pyridinium-containing ring systems that can result when the pyridinium ring is substituted with a C₂ to C₁₀ alkylene or substituted alkylene group includes groups of the formula: 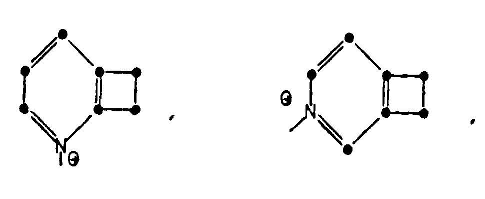 and groups such as: 5H-1-pyrindinium, 7H-1-pyrindinium, 1 H-2-pyrindinium, 5H-2-pyrindinium, thieno[3,2-b]-pyridinium, thieno[3,2-c]pyridinium, thieno[2,3-c]-pyridinium, thieno[2,3-b]pyridinium, thieno[3,4-c]-pyridinium, furo[3,2-b]pyridinium, furo[3,2-c]-pyridinium, furo[2,3-b]pyridinium, furo[3,4-c]-pyridinium, furo-[3,4-b]pyridinium, oxazolo[4,5-b]pyridinium, oxazolo[5,4-b]pyridinium, oxazolo[4,5-c]pyridinium, oxazolo[5,4-c]pyridinium, thiazolo[4,5-b]pyridinium, thiazolo[5,4-b]pyridinium, thiazolo[4,5-c]pyridinium, thiazolo[5,4-c]-pyridinium, 5,6,7,8-tetrahydroquinolinium, 5,6-dihydroquinolinium, 7,8-dihydroquinolinium, 5,6,7,8-tetrahydroisoquinolinium, 5,6-dihydroisoquinolinium, 7,8-dihydroisoquinolinium, 1,5-naphthyridinium, 1,6-naphthyridinium, 1,7-napthyridinium, 1,8-napthyridinium,2,6-napthyridinium, 2,7-napthyridinium, 2H-pyrano-[3,2-c]pyridinium, 5H-pyrano[4,3-b]pyridinium, 1 H-pyrano[3,4-b]pyridinium, 2H-pyrano[2,3-b]pyridinium, 1 H-pyrano[4,3-c]pyridinium, 1 H-pyrano[3,4-c]pyridiniurn, 5H-thiopyrano[4,3-b]pyridinium, 4H-thiopyrano[2,3-b]-pyridinium, pyrido[3,2-d]pyrimidin-5-yl, pyrido[4,3-d]pyrimidin-6-yl, pyrido[3,4-d]pyrimidin-7-yl, pyrido[2,3-d]pyrimidin-8-yl, pyrido[2,3-b]pyrazin-5-yl, pyrido[3,4-b]pyrazin-6-yl, pyrido[2,3-d]pyridazin1-yl, pyrido[3,4-d]pyridazin-6-yl, 4H-pyrido[2,3-d][1,3]oxazin-8-yl, 2H-pyrido[4,3-b][1,4]oxazin-6-yl, 5H-pyrido[2,3-d][1,2]-oxazin-1-yl, 8H-pyrido[3,2-d][1,2]oxazin-1-yl, 1 H-pyrido[2,3-b][1,4]thiazin-5-yl, 3H-pyrido[2,3-b][1,4]-thiazin-5-yl, 2H-pyrido[4,3-b][1,4]thiazin-6-yl, 6,7-dihydro-5H-1-pyrindinium, 6,7-dihydro-5H-2-pyrindinium, 2,3-dihydro-furo[3,2-b]pyridinium, 2,3-dihydro-furo[2,3-b]pyridinium, 2,3-dihydro-thieno[2,3-b]pyridinium, 2,3-dihydro-thieno[3,2-b]pyridinium, 2,3-dihydro-thieno[2,3-c]pyridinium, the substituted derivatives thereof, and the like.

A preferred quaternary ammonium group is a substituted or unsubstituted pyridinium ring.

The substituted pyridinium ring can be substituted once or twice with the above-listed substituents. When the ring is substituted twice, the substituents may be the same or different.

Examples of a group of more particularly preferred substituents on the pyridinium ring are: 3-methyl, 4-methyl, 3-ethyl, 2-ethyl, 4-ethyl, 4-propyl, 2-(isopropyl), 3-(iso-propyl), 2-methyl, 2-(pent-3-yl), 4-(t-butyl), 2-(t-butyl), 2,4-dimethyl, 3,4-dimethyl, 3,5-dimethyl, 2,5-dimethyl, 3-ethyl-4-methyl, 3-methyl-4-ethyl, 3-ethyl-6-methyl, 2-benzyl, 4-benzyl, 2-phenyl, 4-phenyl, 3-phenyl, 2-(hydroxymethyl), 3-(hydroxymethyl), 4-(hydroxymethyl), 3-hydroxy, 2-(1-hydroxyeth-1-yl), 3-(1-hydroxyeth-1-yl), 4-(1-hydroxyeth-1-yl), 3-(2-hydroxyprop-2-yl), 4-(2-hydroxyprop-2-yl), 3-(3-hydroxyprop-1-yl), 3-acetyl, 4-acetyl, 3-benzoyl, 4-benzoyl, 3-methoxy, 4-methoxy, 4-ethoxy, 3-ethoxy, 4-methoxymethyl, 2-methylthio, 4-methylthiomethyl, 3-fluoro, 4-(N-acetamido), 3-ethoxycarbonyl, 4-ethoxycarbonyl, 3-methoxycarbonyl, 3-ethoxycarbonyl, 3-carbamoyl, 4-(N-ethylcarbamoyl), 3-(N,N'-diethylcarbamoyl), 4-cyano, 4-(aminosulfonyl), 4-(potassium eth-1-yl-2-sulfonate), 4-cyclopentyl, 4-(p-chlorobenzyl), 3-alkyl, 5-hydroxy-2-methyl, 3-hydroxy-4-methyl, 4-(1-hydroxypropyl), 3-(1-hydroxypropyl), 3-(2-hydroxy-2-methylpropyl), 2-(hydroxymethyl)-4-methyl, 2-(1,3-di hydroxyprop-2-yl), 4-(2-hydroxypropyl), 4-(3-hydroxy-propyl), 3-cyclohexyl, 4-cyclohexyl, 3-cyclopentyl, 4-(cyclohex-1-enyl), 3-(cyclohex-1-enyl), 4-(cyclopent-1-enyl),3-(cyclopent-1-enyl), 3-(cyclohept-1-enyl), 3-(4-methylcyclohex-1-enyl), 3-(1-hydroxycyclohexyl), 3-(1-hydroxycyclopentyl), 4-(1-hydroxycyclohexyl), 4-(1-hydroxycyclopentyl), 3-(1-hydroxycycloheptyl), 4-methoxy-3-methyl, 3-methoxy-4-methyl, 3-(iso-propoxy), 3-propoxy, 2-(1-methoxyeth-2-yl), 4-(2-ethoxyeth-1-yl), 2-(2-ethoxyeth-1-yl), 4-(acetylmethyl), 4-(3-chloropropyl), 3-(3-chloropropyl), 3-trifluoromethyl, 3-bromo-4-methyl, 3-(cyanomethyl), 4-(1-hydroxy-1-(sulfonic acid)methyl), 4-(cyclopent-2-enyl), 4-(cyclopropyl), and the various protected hydroxy analogs thereof; and a pyridinium ring substituted with the above-described C₂ to C₁₀ alkylene ring, resulting in the following bicyclic ring examples: 5,6-dihydro-5H-1-pyrindinium, 5,6,7,8-tetrahydroquinolinium, 5,6,7,8-tetrahydroisoquinolinium, 3-methyl-5,6,7,8-tetrahydroquinolinium, 6,7-dihydro-5H-2-pyrindinium, 7-hydroxy-5,6-dihydro-5H-1-pyrin- dinium, 5,6,8,9-tetrahydro-7H-cyclohepta[b]pyridinium, 2,3-dihydrofuro[2,3-b]pyridinium, 3-hydroxy-2,3-dihydro-furo[2,3-b]pyridinium, 3-keto-2,3-dihydro-furo[2,3-b]pyridinium, thieno[3,2-b]pyridinium, thieno[3,2-c]pyridinium, furo[3,2-c]pyridinium, 2-methylthiazolo[4,5-c]pyridinium, and 2-methylthiazolo[5,4-c]pyridinium.

A preferred group of substituted pyridinium rings are 4-carbamoylpyridinium, 4-(2-(sulfoethyl)-pyridinium, 5,6-dihydro-5H-1-pyrindinium, thieno[3,2-b]-pyrindinium, thieno[3,2-c]pyridinium, furo[3,2-c]-pyridinium, 2-methylthieno[4,5-c]pyridinium and 2-methylthieno[5,4-c]pyridinium.

A more preferred group of pyridinium rings is pyridinium, 4-carbamoylpyridinium, 4-(sodium eth-1-yl-2-sulfonate)pyridinium, 5,6-dihydro-5H-1-pyrindinium, 2-methylthiazolo[4,5-c]pyridinium and 2-methylthiazolo-[5,4-c]pyridinium.

Another preferred quaternary ammonium group is the substituted or unsubstituted quinolinium group. The quinolinium group may be substituted on the A or B ring or on both rings with the same or different substituents. Some examples and description of substituted quinolinium groups can be found in W.H.W. Lunn, U.S. Patent No. 4,396,620, issued August 2, 1983, herein incorporated by reference. Columns 3, 4, 13, 14, 15, 16, 17, 18, 19 and 20 of the '620 patent are particularly helpful in this regard.

A preferred group of quinolinium groups are the quinolinium, 5-aminoquinolinium, 3-aminoquinolinium, 2-aminoquinolinium, 7-aminoquinolinium, 5-hydroxyquinolinium, 6-hydroxyquinolinium and 7-hydroxyquinolinium group.

Another preferred quaternary ammonium group is the substituted or unsubstituted isoquinolinium group. The isoquinolinium ring may be substituted on the A or the B ring or on both rings with the same or different substituents.

Examples and description of substituted isoquinolinium groups can be found in W.H.W. Lunn, U.S. Patent No. 4,396,619, issued August 2, 1983, herein incorporated by reference. Columns 3, 4, 13, 14, 16, 17, 18, 19, 20, 21 and 22 of the '619 patent are particularly helpful in this regard.

A preferred group of isoquinolinium substituents are isoquinolinium, the hydroxy-substituted isoquinolinium groups such as 5-hydroxyisoquinolinium or 4-hydroxyisoquinolinium, or the amino-substituted isoquinolinium groups such as 4-aminoisoquinolinium, 5-aminoisoquinolinium or 6-aminoisoquinolinium.

A more preferred group of isoquinolinium groups are the isoquinolinium, 5-aminoisoquinolinium and 8-hydroxyisoquinolinium groups.

Another preferred quaternary ammonium group is a 1-pyridazinium or 2-pyridazinium group, or a mono-or di-substituted analog thereof, wherein the substituents can be the same or different.

A preferred group of pyridazinium substituents include: pyridazinium (unsubstituted), 3,6-dich- loropyridazinium, 3-methylpyridazinium, 3,6-di(hydroxy)-pyridazinium, 3-chloro-6-methoxypyridazinium, 3,5-di(hydroxy)pyridazinium, 4-methylpyridazinium, 3-methoxypyridazinium, 4-methoxypyridazinium, 3,6-dimethylpyridazinium, 3-(methylthio)pyridazinium, 4-(methylthio)pyridazinium, 3-aminopyridazinium, 4-aminopyridazinium, 3-amino-6-methylpyridazinium, 3,6-di(methoxy)pyridazinium, 6-aminopyridazinium, 6-(methylamino)pyridazinium, 6-chloro-3-methoxypyridazinium, 5-methylpyridazinium, and 5-ethyl- pyridazinium.

A more preferred pyridazinium group is unsubstituted pyridazinium.

Another preferred quaternary ammonium group is a 1-pyrimidinium or 3-pyrimidinium group, or a mono- or di-substituted analog thereof, wherein the substituents can be the same or different. A preferred group of pyrimidinium substituents is 4,5-diaminopyrimidinium, 4,6-diaminopyrimidinium, the (protected amino)pyrimidinium analogs thereof, 4-phenylpyrimidinium, 4,6-dichloropyrimidinium, 2,4-dichloropyrimidinium, 4,6-di(methyl)pyrimidinium and the unsubstituted pyrimidinium group. A more preferred pyrimidinium group is unsubstituted pyrimidinium.

Another preferred quaternary ammonium group is the pyrazinium group, or a mono- or di-substituted analog thereof, wherein the substituents can be the same or different. A preferred group of pyrazinium substituents include 3-methylpyrazinium, 3,5-di(methyl)pyrazinium, 3-aminopyrazinium, 3-protected aminopyrazinium, 3-ethyl-pyrazinium, 3-(diethylamino)pyrazinium, 3-(ethylamino)-pyrazinium, 3,5-diethylpyrazinium, 3-(dimethylamino)-pyrazinium, 2,6-dimethylpyrazinium, 2-chloropyrazinium, 3-chloropyrazinium, 2-aminopyrazinium, 2-carboxy-3-aminopyrazinium, 2,6-dichloropyrazinium, 2,3-dimethylpyrazinium, 2,5-dimethylpyrazinium, 2-methylpyrazinium, 2-carbamoylpyrazinium, 2-carboxypyrazinium, 2,3-dicarbamoyl- pyrazinium, 2,3-dicarboxypyrazinium, 2-methylpyrazinium, 2-ethylpyrazinium, 2-ethyl-3-methyl-pyrazinium, 2-ethyl-5-methylpyrazinium, 2-ethyl-6-methylpyrazinium, 2,5-diethylpyrazinium, 3-(iso-propyl)-2-ethox- ypyrazinium, 3-(sec-butyl)-2-methoxypyrazinium and 3-(iso-butyl)-2-methoxypyrazinium. A more preferred group of pyrazinium substituents is the unsubstituted pyrazinium and the 2-(dimethylamino)pyrazinium-1-yl groups.

Another group of preferred quaternary ammonium substituents is the substituted or unsubstituted thiazolinium, isothiazolinium, oxazolinium, isoxazolinium, N3-(C, -C4 alkyl) imidazolinium, 1,3,4-thiadiazolinium, 1,2,4-thiadiazolinium, 1,2,5-thiadiazolinium, 1,3,4-oxadiazolinium, 1,2,4-oxadiazolinium or 1,2,5-oxadiazolinium groups, each of which can be substituted once or twice with the same or different substituents. The groups containing two nitrogen atoms in the ring may be quaternized at either ring nitrogen.

A preferred ring system in this group is the substituted or unsubstituted thiazolium group. A preferred thiazolium group is the 4-methyl-5-(1-hydroxyeth-2-yl)-thiazolium ring.

Also, a preferred quaternary ammonium group is a group of quaternary ammonium substituents which have the formula: 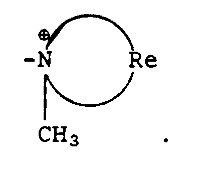 The preferred compounds of the above quaternary ammonium group occur when the variable Re, taken together with the nitrogen atom to which it is bonded, represents a saturated or mono-unsaturated 5-, 6-, 7-or 8-membered heterocyclic ring optionally containing a further nitrogen or oxygen heteroatom. The heterocyclic ring may be mono-substituted and may also be fused with a benzene ring.

A preferred substituent within this quaternary ammonium group is of the formula 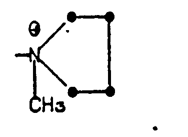 Further examples and description of this type of quaternary ammonium group can be found in P.E. Ayres, U.S. Patent No. 4,168,309, issued September 18, 1979, herein incorporated by reference.

The term "heterocyclic ring" denotes optionally substituted five-membered or six-membered rings that have 1 to 4 heteroatoms, such as oxygen, sulfur and/or nitrogen, in particular nitrogen, either alone or in conjunction with sulfur or oxygen ring atoms. These five-membered or six-membered rings may be fully unsaturated or partially unsaturated, with fully unsaturated rings being preferred.

Furthermore, the above optionally substituted five-membered or six-membered rings can optionally be fused to a aromatic 5-membered or 6-membered ring system. For example, the rings can be optionally fused to an aromatic 5-membered or 6-membered ring system such as a pyridine or a triazole system, and preferably to a benzene ring.

The following ring systems are examples of the heterocyclic (whether substituted or unsubstituted) radicals denoted by the term "heterocyclic ring": thienyl, furyl, pyrrolyl, imidazolyl, pyrazolyl, thiazolyl, isothiazolyl, oxazolyl, isoxazolyl, triazolyl, thiadiazolyl, oxadiazolyl, tetrazolyl, thiatriazolyl, oxatriazolyl, pyridyl, pyrimidyl, pyrazinyl, pyridazinyl, thiazinyl, oxazinyl, triazinyl, thiadiazinyl, oxadiazinyl, dithiazinyl, dioxazinyl, oxathiazinyl, tetrazinyl, thiatriazinyl, oxatriazinyl, dithiadiazinyl, imidazolinyl, dihydropyrimidyl, tetrahydropyrimidyl, tetrazolo[1,5-b]pyridazinyl and purinyl, as well as benzo-fused derivatives, for example benzoxazolyl, benzthiazolyl, benzimidazolyl and indolyl.

A preferred group of the above heterocyclic rings are 5-membered ring systems containing a sulfur or oxygen atom and one to three nitrogen atoms. Examples of such preferred groups include thiazolyl, in particular thiazol-2-yl and thiazol-2-yl N-oxide, thiadiazolyl, in particular 1,3,4-thiadiazol-5-yl and 1,2,4-thiadiazol-5-yl, oxazolyl, preferably oxazol-2-yl, and oxadiazolyl, such as 1,3,4-oxadiazol-5-yl and 1,2,4-oxadiazol-5-yl. A group of further preferred examples of 5-membered ring systems with 2 to 4 nitrogen atoms include imidazolyl, preferably imidazol-2-yl; triazolyl, preferably 1,3,4-triazol-5-yl, 1,2,3-triazol-5-yl, and 1,2,4-triazol-5-yl, and tetrazolyl, preferably 1 H-tetrazol-5-yl. A preferred group of examples of benzo-fused derivatives are, in particular, benzoxazolyl-2-yl, benzthiazol-2-yl and benzimidazol-2-yl.

Further specific examples of the above heterocyclic ring systems are 6-membered ring systems containing one to three nitrogen atoms. Such examples include pyridyl, such as pyrid-2-yl, pyrid-3-yl and pyrid-4-yl; pyrimidyl, preferably pyrimid-2-yl and pyrimid-4-yl; triazinyl, preferably 1,3,4-triazin-2-yl and 1,3,5-triazin-4-yl; pyridazinyl, in particular pyridazin-3-yl, and pyrazinyl. The pyridine N-oxides and pyridazine N-oxides, and the pyridyl, pyrimid-2-yl, pyrimid-4-yl, pyridazinyl and the 1,3,4-triazin-2-yl radicals, are a preferred group.

The substituents for the optionally substituted heterocyclic ring systems, and further examples of the 5-and 6- membered ring systems discussed above, are found in W. Durckheimer et al., U.S. Patent No. 4,278,793, issued July 14, 1981, columns 9 through 21 and columrs 33 through 188, herein incorporated by reference. (In columns 33 through 188, examples of the term "heterocyclic ring" are included in the heterocyclic thiomethyl groups listed under heading "A".)

A particularly preferred group of examples of the term "heterocyclic ring" is 1,3-thiazol-2-yl, 4-(carboxymethyl)-5-methyl-1,3-thiazol-2-yl, 4-(carboxy-methyl)-5-methyl-1,3-thiazol-2-yl sodium salt, 1,2,4-thiadiazol-5-yl, 3-methyl-1,2,4-thiadiazol-yl-5-yl, 1,3,4-triazol-5-yl, 2-methyl-1,3,4-triazol-5-yl, 2-hydroxy-1,3,4-triazol-5-yl, 2-carboxy-4-methyl-1,3,4-triazol-5-yl sodium salt, 2-carboxy-4-methyl-1,3,4-triazol-5-yl, 1,3-ox- azol-2-yl, 1,3,4-oxadiazol-5-yl, 2-methyl-1,3,4-oxadiazol-5-yl, 2-(hydroxymethyl)-1,3,4-oxadiazol-5-yl, 1,2,4-oxadiazol-5-yl, 1,3,4-thiadiazol-5-yl, 2-methyl-1,3,4-thiadiazol-5-yl, 2-thiol-1,3,4-thiadiazolyl-5-yl, 2-methylthio)-1,3,4-thiadiazol-5-yl, 2-amino-1,3,4-thiadiazol-5-yl, 1 H-tetrazol-5-yl, 1-methyl-1 H-tetrazol-5-yl, 1-(1-(dimethy!amino)eth-2-y!)-1 H-tetrazol-5-yl, 1-(carboxymethyl)-1H-tetrazol-5-yl, 1-(carboxymethyl)-1H-tetrazol-5-yl sodium salt, 1-(methylsulfonic acid)-1 H-tetrazol-5-yl, 1-(methylsulfonic acid)-1 H-tetrazol-5-yl sodium salt, 2-methyl-1 H-tetrazol-5-yl, 1,2,3-triazol-5-yl, 1-methyl-1,2,3-triazol-5-yl, 2-methyl-1,2,3-triazol-5-yl, 4-methyl-1,2,3-triazol-5-yl, pyrid-2-yl N-oxide, 6-methoxy-2-(N-oxide)-pyridaz-3-yl, 6-hydroxypyridaz-3-yl, 1-methylpyrid-2-yl, 1-methylpyrid-4-yl, 2-hydroxypyrimid-4-yl, 1,4,5,6-tetrahydro-5,6-dioxo-4-methyl-as-triazin-3-yl, 1,4,5,6-tetrahydro-4-(formylmethyl)-5,6-dioxo-as-triazin-3-yl, 2,5-dihydro-5-oxo-6-hydroxy-as-triazin-3-yl, 2,5-dihydro-5-oxo-6-hydroxy-as-triazin-3-yl sodium salt, 2,5-dihydro-5-oxo-6-hydroxy-2-methyl-as-triazin-3-yl sodium salt, 2,5-dihydro-5-oxo-6-hydroxy-2-methyl-as-triazin-3-yl, 2,5-dihydro-5-oxo-6-methoxy-2-methyl-as- triazin-3-yl, 2,5-dihydro-5-oxo-as-triazin-3-yl, 2,5-dihydro-5-oxo-2-methyl-as-triazin-3-yl, 2,5-dihydro-5-oxo-2,6-dimethyl-as-triazin-3-yl, tetrazolo[1,5-b]pyridazin-6-yl and 8-aminotetrazolo[1,5-b]pyridazin-6-yl.

A further preferred group of 1-carbacephalosporins is represented by the formula I wherein R is the substituted methyl group 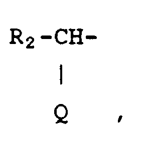 particularly those compounds wherein Q is amino or substituted amino. Especially preferred compounds are represented when Q is amino. Examples of such substituents, together with the CO moiety to which they are attached, are D-phenylglycyl, D-4-hydroxyphenylglycyl, D-2-thienylglycyl, D-benzothien-3-ylglycyl, and like functionalities.

A further preferred group is represented by formula I wherein R is the group 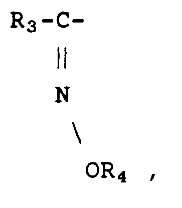 in the syn form.

Particularly preferred compounds are represented when R₄ is C₁ -C₄ alkyl or a carboxy substituted alkyl group such as carboxymethyl, 2-carboxyethyl, 3-carboxypropyl, and 2-carboxy-2-propyl; and R₃ is a five or six membered heterocyclic ring Rᵢ, in particular, an amino substituted heterocyclic. Especially preferred heterocyclics are the 2-aminothiazole or 2-aminooxazole ring. Examples of such preferred RCO-groups are (2-aminothiazol-4-yl)(methoxyimino)acetyl, (2-aminooxazol-4-yl)(methoxyimino)acetyl, and the like.

Further preferred compounds of this group are represented by the formula 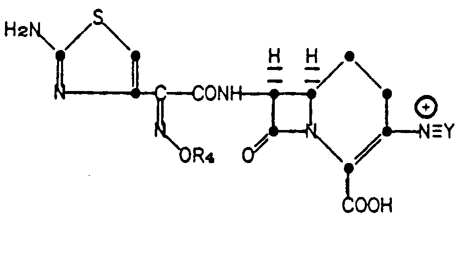 and the pharmaceutically acceptable non-toxic salts thereof. Preferred structures are those when -N=Y is substituted or unsubstituted pyridinium, quinolinium, isoquinolinium, 1- or 2-pyridazinium, 1- or 3- pyrimidinium, pyrazinium, imidazolinium, thiazolinium, isothiazolinium, oxazolinium, isoxazolinium, 1,2,4-, 1,2,5-, or 1,3,4-thiadiazolinium or -oxadizolinium, or a group of the formula

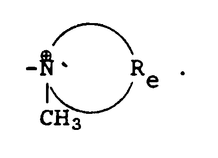

The term "pharmaceutically acceptable salt" refers to salts of the carboxy group or other acidic moiety in the molecule, such as a carboxy or sulfo substituent group, and includes salts formed with organic amines and inorganic bases. Such amines and bases include those whose counter-ions are chosen from the alkali and alkaline earth metals (such as lithium, sodium, potassium, barium and calcium); ammonium; and the organic cations (such as dibenzylammonium, benzylammonium, 2-hydroxyethylammonium, bis(2-hydroxyethyl)-ammonium, phenylethylbenzylammonium, dibenzylethylenediammonium, and like cations). Other cations encompassed by the above term include the protonated form of procaine, quinine and N-methylglucosamine, and the protonated forms of basic amino acids such as glycine, ornithine, histidine, phenylglycine, lysine and arginine. Furthermore, any zwitterionic form of the instant compounds formed by a carboxylic acid and an amino group is referred to by this term. For example, a cation for a carboxylate anion will exist in the presence of the quaternary ammonium group. A preferred cation for the carboxylate anion is the sodium cation.

Furthermore, the term includes salts that form by standard acid-base reactions with basic groups of the compounds of this invention (such as amino groups) and organic or inorganic acids. Such acids include hydrochloric, sulfuric, phosphoric, acetic, succinic, citric, lactic, maleic, fumaric, palmitic, cholic, pamoic, mucic, D-glutamic, d-camphoric, glutaric, phthalic, tartaric, lauric, stearic, salicyclic, methanesulfonic, benzenesulfonic, sorbic, picric, benzoic, cinnamic, and like acids.

The compounds of Formula I may also exist as solvates. Thus, these compounds may crystallize with, for example, waters of hydration, or one, a number of, or any fraction thereof of molecules of the mother liquor solvent, such as ethanol. The solvates of such compounds are included within the scope of this invention.

Furthermore, the compounds of Formula I encompass the requisite negative counter-ion of the quaternary ammonium group. Such a counter-ion may be a carboxylate anion, an anionic group bound at some other place to the molecule or a separate external counter-ion such as a halo or acyloxy anion.

The 3-quaternary ammonium compounds of this invention are prepared by reacting the corresponding 3-triflate ester with the appropriate amine N=Y. The reaction may be performed on any of the protected or otherwise functionalized triflate esters but is preferably the last or penultimate step in a series designed to introduce the other desired fuctionalities into the molecule, such as the desired acyl group of the 7-position. Moreover, blocking groups, both on the 4-carboxy moiety and which protect any acid or amine groups on the 7-substituent, are preferably introduced before the conversion of the triflate group into the desired quaternary ammonium functionality. The blocking groups can then be removed sequentially, or in some cases, such as in a preferred embodiment in which Rₐ is allyl and the protecting group on the 7-side chain is allyloxycarbonyl, can be removed under the same conditions. Other transformations, such as salt formation, conversion to a biological labile ester, etc., can be carried out thereafter.

A typical series of transformations is summarized in Scheme I below: 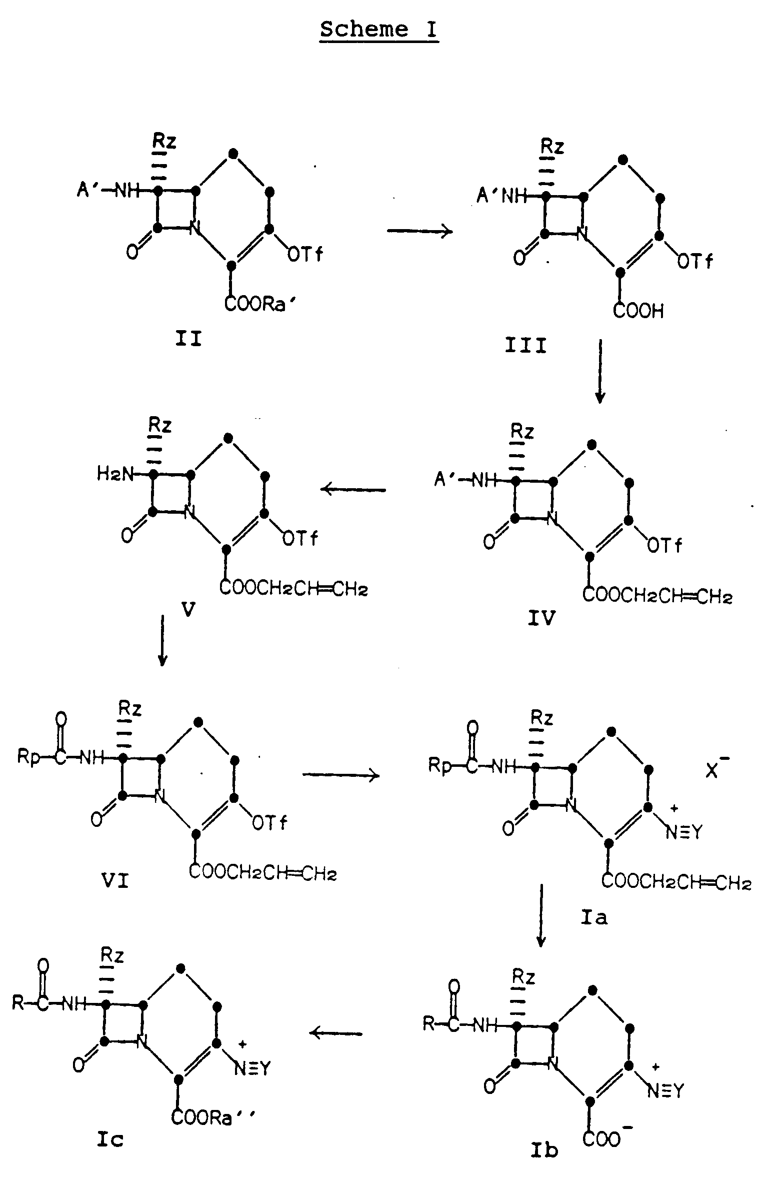 wherein A' is an amino protecting group, Tf is a triflate moiety (-S0₂CF₃), Ra is a carboxy protecting group, such as a p-nitrobenzyl moiety, Rp is R or a protected R group, and Rₐ" is a biologically labile group.

While this scheme employs a specific acid protecting group, i.e., the allyl ester of the 4-carboxy group, it will be apparent that any other suitable protecting groups as described earlier can be employed with the corresponding changes in the deprotection step as will be appreciated by those skilled in the art.

According to Scheme I, a triflate ester of Formula II can, if necessary, be hydrolyzed to prepare the corresponding acid III and then converted to a protected ester IV which is most suitable for the subsequent transformations. Hydrolysis of the carboxy protecting group followed by protection of the carboxylic acid with another protective group can be accomplished by employing procedures that are well known in the cephalosporin, penicillin, and peptide arts.

Examples of these groups are found in T. W. Green, "Protective Groups in Organic Synthesis," John Wiley and Sons, New York, NY, 1981.

Similarly, the amino protecting group (or any other acyl group of the 7-position) on the amine of IV can be removed and replaced with the desired side chain RCO or a protected version thereof.

The transformation of the suitable substituted and protected triflate ester VI to the corresponding quaternary ammonium derivative la is accomplished by allowing VI to react with a molar excess of the amine N=Y. In cases where the amine is a liquid, the reaction is best carried out by using the amine both as reactant and solvent. The reaction is carried out by simply stirring the amine and triflate ester at temperatures from approximately 0_{°}C to about 100_{°}C, preferably 20-30 °C. The reaction is usually complete in 1-3 days. Alternatively, an inert solvent, such as acetonitrile, can be used in addition to the above reagents.

The quaternary ammonium compounds la, or other acid protected derivatives thereof, can then be deprotected to provide the carboxylic acids of this invention, Formula lb. In certain cases, the deprotection will remove both the carboxy protecting group and a protecting group on the 7-side chain. In the preferred situation where the allyloxycarbonyl group is used to protect an amino group in the 7-position side chain or the 7-amino group, both this group and the allyl ester can be removed upon treatment with triphenyl phosphine, palladium acetate, and tributyl tin hydride in solvents such as acetonitrile and diethyl ether, as taught by O. Dangles, F. Guibe, and G. Balovoine, J. Org. Chem., 52, 4984 (1987) and L. N. Jungheim and S. K. Sigmund, J. Org. Chem., 52, 4007 (1987). Alternatively, the allyl or other ester group may be removed separately from a side chain protecting group in a sequence appropriate for the selective removal of the respective protecting groups.

If a labile ester product is desired, Ib can be transformed into the corresponding derivative Ic by standard methods.

The 7β-acylamino-7α-substituted-1-carbacephalosporins represented by the formula I wherein R_{z} is C₁-C₄ alkoxy, or precursors thereto, are prepared according to the method described by Koppel, U.S. Patent No. 3,994,885. The 7a-formamido substituted compounds wherein R_{z} is -NHCHO are obtained by the method described by Millner, U.S. Patent No. 4,539,159. According to this method, a 7β-acylamino- or 7β-protectedamino-7-a-methylthio-substituted 1-carbacephalosporin is reacted with anhydrous ammonia or an ammonium salt in the presence of mercuric acetate to form the corresponding 7a-amino derivative. The latter is formylated to the 7a-formamido derivative.

The starting material II is available according to the disclosure and procedure of EPO Patent Application No. 211,540. These triflate derivatives are prepared from the corresponding 3-hydroxy compounds reported in EPO Patent Application No. 209,352. Other reagents and intermediates required for preparing the compounds of this invention are commercially available, reported in the literature, or can be prepared by conventional methods known in the art. For example, the triflate esters may be prepared from, or converted into, the corresponding 3-mesyl, -tosyl, -phosphonate, or -halo congener by standard methods. These derivatives can therefore serve as intermediates to the 3-triflates or as derivatives of the triflates which can be converted into the 3-quaternary compounds of this invention according to the methods described above for the 3-triflate intermediates.

The following examples further illustrate the preparation of the starting materials, intermediates, and compounds of this invention. The examples are illustrative only and are not intended to limit the scope of the invention in any way. The term "m/e" used in characterizing the products refers to the mass-to-charge ratio of ions which appear in the mass spectra of the products. In general, the values correspond to molecular weights of the major peaks, and are so designated "M⁺". Where structures were confirmed by mass spectral, infrared, or proton magnetic resonance analysis, the compound is so designated by "MS", "IR", or "NMR", respectively, in the solvent as indicated. NMR values are reported as parts per million (ppm).

### Example 1

### 1-[7,β-{[2-(allyloxycarbonylamino)-4-thiazolyl](methoxyimino)acetyl}amino)-2-(allyloxycarbonyl)-8-oxo-1-azabicyclo[4.2.0]oct-2-en-3-yl]pyridinium trifluoromethanesulfonate

### A. Preparation of p-nitrobenzyl 7β-phenoxyacetylamino-3-trifluoromethylsulfonyloxy-1-carba(dethia)-3-cephem-4-carboxylate.

A solution of 4.59 g of p-nitrobenzyl 7β-phenoxyacetylamino-3-hydroxy-1-carba(dethia)-3-cephem-4-carboxylate in 98 ml of distilled methylene chloride was cooled to -40 °C by means of an external acetonitrile/dry ice bath. With stirring, 1.49 g of triethylamine were added followed by the rapid addition of 2.91 g of triflic anhydride. After 5 minutes, the reaction mixture was poured into a saturated aqueous sodium bicarbonate solution. The layers were separated and the organic layer was washed twice with 0.1 N hydrochloric acid, once with distilled water, then dried over magnesium sulfate, filtered, and concentrated in vacuo. Hexane was added and the mixture again evaporated in vacuo. The residue was triturated with 100 ml of hexane providing 5.5 g of the desired subtitle intermediate as light pink/beige crystals.
m/e: M⁺ + 1 = 600
NMR (CDCI₃): 1.50-2.18 (2H, m), 2.46-2.74 (2H, m), 3.82-4.10 (1H, m), 4.50 (3H, s), 5.30-5.54 (3H, m), 6.78-7.58 (6H, m), 8.18 (2H, d).

### B. Preparation of 7β-phenoxyacetylamino-3-trifluoromethanesulfonyloxy-1-carba(dethia)-3-cephem-4-carboxylic acid.

A mixture of 5.30 g of the ester of Example 1A above, 135 ml of tetrahydrofuran, and 135 ml of dimethylformamide was cooled by means of an external ice/ethanol bath. Under a nitrogen atmosphere, 135 ml of 1N hydrochloric acid were added followed by the addition of 2.20 g of zinc powder. After stirring for two hours at 0 °C, the mixture was poured into 2 liters of ethyl acetate, washed twice with 400 ml of 1N hydrochloric acid and once with 400 ml of water, dried over magnesium sulfate, filtered, and concentrated in vacuo providing 4.2 g of of the desired subtitle acid which was used in the subsequent step without further purification.

### C. Preparation of allyl 7β-phenoxyacetylamino-3-trifluoromethylsulfonyloxy-1-carba(dethia)-3-cephem-4-carboxylate.

The 4.2 g of acid intermediate from Example 1B above was suspended in 100 ml of a saturated sodium bicarbonate solution. To the suspension were added 3.19 g of tetrabutylammonium hydrogen sulfate followed by the addition of 50 ml of methylene chloride. After stirring for 10 minutes, the mixture was extracted 4 times with methylene chloride. The combined organic extracts were dried over sodium sulfate, filtered, and concentrated in vacuo. The resulting oil was dissolved in 10 ml of chloroform to which 1.307 g of allyl bromide were added. The reaction mixture was stirred under a nitrogen atmosphere overnight. The mixture was then concentrated in vacuo and chromatographed over silica gel eluting with 50:50 ethyl acetate/hexane. The appropriate fractions were combined and concentrated in vacuo, providing 3.0 g of the desired title intermediate as a clear oil.
NMR (CDCI₃): 1.84-2.24 (2H, m), 2.52-2.76 (2H, m), 3.76-4.12 (1 H, m), 4.50 (1 H, s), 4.76 (2H, 2d), 5.16-5.56 (4H, m), 5.68-6.20 (2H, m), 6.76-7.56 (6H, m).

### D. Preparation of allyl 7β-amino-3-trifluoromethylsulfonyloxy-1-carba〈dethia〉-3-cephem-4-carboxylate.

To a solution of 2.6 g of the allyl ester from Example 1 C above in 52 ml of methylene chloride were added 0.50 g of pyridine followed by 1.21 g of phosphorus pentachloride. After stirring at room temperature for over 1 hour, an additional 0.020 g of pyridine and 0.054 g of phosphorus pentachloride were added. After stirring an additional 30 minutes, the reaction mixture was added to a solution of 3.85 g of isobutyl alcohol and 215 ml of methylene chloride which had previously been cooled to -20 °C. After stirring for 90 minutes, 150 ml of water were added and the mixture stirred vigorously for 30 minutes. The layers were separated and the organic layer washed twice with 1 N hydrochloric acid. The aqueous layers were combined, adjusted to pH 7.5, and extracted 4 times with chloroform. The chloroform extracts were combined, dried over sodium sulfate, filtered, and concentrated in vacuo. The resulting clear oil contained the title intermediate which was used in the subsequent acylation step without further purification.

### E. Preparation of allyl 7β-({[2-(allyloxycarbonyl)amino-4-thiazolyl](methoxyimino)acetyl}amino)-3-trifluoromethylsulfonyloxy-1-carba(dethia)-3-cephem-4-carboxylate.

Under a nitrogen atmosphere, 1.04 g of 2-{[(allyloxycarbonyl)amino]-4-thiazolyl}(methoxyimino)acetic acid were suspended in 37 ml of methylene chloride. After cooling by means of an external ice/ethanol bath, 0.52 g of N-methylmorpholine were added to the reaction mixture followed by the introduction of a solution of 0.90 g of 2-chloro-4,6-dimethoxy-1,3,5-triazine in 2 ml of methylene chloride. After stirring at 0 °C for 30 minutes, the reaction was stirred an additional 45 minutes at room temperature. The solution was cooled by means of the external ice/ethanol bath and the solution of the crude free amine from Example 1 D above in 15 ml of methylene chloride was added in dropwise fashion. After stirring at room temperature overnight, the resulting solid was removed by filtration and the filtrate concentrated in vacuo. The residue was dissolved in ethyl acetate, washed successively with 2 portions of 1N hydrochloric acid, once with water, twice with a saturated sodium bicarbonate solution, and once with water. The organic layer was dried over magnesium sulfate, filtered, and concentrated in vacuo. The resulting oil was chromatographed over silica gel eluting with 60:40 ethyl acetate/hexane. The appropriate fractions were combined and concentrated in vacuo providing a white solid. The residue was triturated with diethyl ether to provide 975 mg of the desired title intermediate.
m/e: M⁺ + 1 = 638
IR (CDCl₃): 1774 cm―¹ (β-lactam carbonyl)
NMR (CDCl₃): 1.83-2.00 (1H, m), 2.18-2.30 (1H, m), 2.62-2.74 (2H, m), 4.0 (3H, s), 4.01-4.10 (1H, m), 4.64-4.84 (4H, 2d), 5.28-5.44 (4H, m), 5.62-5.68 (1H, m), 5.90-6.02 (2H, m), 7.14 (1 H, s), 7.96 (1H, d), 9.50 (1 H, s).

### F. Preparation of 1-[7β-{[2-(allyloxycarbonylamino)-4-thiazolyl](methoxyimino)acetyl}amino)-2-allyloxycarbonyl)-8-oxo-1-azabicyclo[4.2.0]oct-2-en-3-yl]pyridinium trifluoromethanesulfonate.

One gram of the 3-triflate ester named in Example 1E above was dissolved in 0.8 ml of acetonitrile under a nitrogen atmosphere. To this solution was introduced 0.14 ml of pyridine. The reaction mixture was stirred under a nitrogen atmosphere for 3 days having added 0.14 ml and 0.07 ml of pyridine after 1 and 2 days, respectively. The mixture was concentrated in vacuo, redissolved in acetonitrile, and again evaporated. The residue was chromographed over silica gel eluting with a gradient of 50% 25:9:7:9 ethyl acetate/acetonitrile/acetic acid/water in ethyl acetate to 100% 25:9:7:9 ethyl acetate/acetonitrile/acetic acid/water. The desired fractions were combined, concentrated in vacuo, and then lyophilized providing 0.95 g of the desired title intermediate as a dark orange solid.
NMR (CD₂CI₂): 3.96 (3H, s), 4.08-4.24 (1H,m), 4.54 (2H, d), 4.72 (2H, d), 5.04-6.20 (7H, m), 7.20 (1H, s), 7.92-8.20 (2H, m), 8.32-8.60 (1 H, m), 9.12-9.36 (2H, m).

### Examples 2-10

The following intermediates were prepared according to the procedure of Example 1 F from the intermediate of Example 1 E or related intermediate and the corresponding pyridine or imidazole derivative.

### 2. 6-[7β-({[2-(allyloxycarbonyl)amino-4-thiazolyl](methoxyimino)acetyl}amino-2-(allyloxycarbonyl)-8-oxo-1-azabicyclo[4.2.0]oct-2-en-3-yl]-2-(4-methoxyphenyl)thieno[2,3-c]pyridinium trifluoromethanesulfonate, 35% yield.

NMR (CDCI₃): 3.84 (6H, s), 4.04-4.28 (1H,m), 4.36-4.76 (4H, 2d), 4.88-5.20 (9H, m), 6.88-7.06 (2H, m), 7.12 (1 H, s), 7.60-7.80 (3H, m), 7.88-8.16 (1 H, m), 8.36-8.64 (1 H, m).

### 3. 3-[7β-({[2-(allyloxycarbonyl)amino-4-thiazolyl](methoxyimino)acetyl}amino)-2-(allyloxycarbonyl)-8-oxo-1-azabicyclo[4.2.0]oct-2-en-3-yl]-1-methyl-1 H-imidazolium trifluoromethanesulfonate, 77% yield.

NMR (DMSO): 1.73-1.93 (1 H, m), 2.10-2.23 (1 H, m), 2.50-2.77 (1 H, m), 2.80-2.97 (1 H, m), 3.87 (3H, s), 3.89 (3H, s), 3.93-4.13 (1 H, m), 4.53-4.70 (4H, m), 5.13-5.50 (4H, m), 5.63-5.83 (2H, m), 5.87-6.03 (1 H, m), 7.37 (1 H, s), 7.83 (1 H, s ), 7.97 (1 H, s), 9.35 (1 H, s), 9.52 (2H, d).

### 4. 5-[7β-({[2-(allyloxycarbonyl)amino-4-thiazolyl](methoxyimino)acetyl}amino)-2-(allyloxycarbonyl)-8-oxo-1-azabicyclo[4.2.0]oct-2-en-3-yl]-3-methyl-3H-imidazo[4,5-c]pyridin-5-ium trifluoromethanesulfonate, 71% yield.

NMR (CD₃CN): 2.05-2.41 (2H, m), 3.89 (3H, s), 4.01 (3H, s), 4.05-4.27 (1H, m), 4.43 (2H, d), 4.63 (2H, d), 4.89-6.13 (8H, m), 7.13 (1 H, s), 8.01 (1 H, d), 8.43 (1 H, d), 8.57 (1 H, s).

### 5. 1-[7β-({[2-(allyloxycarbonyl)amino-4-thiazolyl](methoxyimino)acetyl}amino)-2-(allyloxycarbonyl)-8-oxo-1-azabicyclo[4.2.0]oct-2-en-3-yl]-6,7-dihydro-5H-1-pyrindinium trifluoromethanesulfonate, 75% yield.

NMR (CD₃CN): 2.00-2.32 (4H, m), 2.68-3.00 (2H, m), 3.04-3.50 (4H, m), 3.92 (3H, s), 4.04-4.28 (1H, m), 4.62 (2H, d), 4.66 (2H, d), 5.04-5.32 (4H, m), 5.38-5.68 (2H, m), 5.72-6.04 (1 H, m), 7.28 (1 H, s), 7.40-7.82 (2H, m), 8.16-8.32 (2H, m).

### 6. 1-[7β-({[2-(allyloxycarbonyl)amino-4-thiazolyl](methoxyimino)acetyl}amino)-2-(allyloxycarbonyl)-8-oxo-1-azabicyclo[4.2.0]oct-2-en-3-yl]-5,6,7,8-tetrahydroquinolinium trifluoromethanesulfonate, 65% yield. NMR.

### 7. 1-[7β-({[2-(allyloxycarbonyl)amino-4-thiazolyl](methoxyimino)acetyl}amino)-2-(allyloxycarbonyl)-8-oxo-1-azabicyclo[4.2.0]oct-2-en-3-yl]-2,5-dimethylpyridinium trifluoromethanesulfonate, 65% yield.

m/e: M⁺ + 1 = 595
NMR (CD₃CN): 1.87-2.93 (10H, m), 4.0 (3H, s), 4.17-4.25 (1H, m), 4.23-4.77 (4H, m), 5.17-5.47 (4H, m), 5.60-5.80 (2H, m), 5.93-6.09 (2H, m), 7.30 (1 H, s), 7.58 (1 H, d), 7.95 (1 H, d), 8.32 (1 H, d), 8.40 (1 H, s), 9.43 (1 H, s).

### 8. 1-[7β-({[2-(allyloxycarbonyl)amino-4-thiazolyl](methoxyimino)acetyl}amino)-2-(allyloxycarbonyl)-8-oxo-1-azabicyclo[4.2.0]oct-2-en-3-yl]-3,4-dimethyl-pyridinium trifluoromethanesulfonate, 94% yield.

NMR (CD₃CN): 1.87-2.33 (2H, m), 2.45 (3H, s), 2.60 (3H, s), 2.80-2.88 (2H, m), 4.00 (3H, s), 4.13-4.23 (1 H, m), 4.27-4.60 (2H, m), 4.75 (2H, d), 5.17-5.47 (4H, m), 5.63-5.77 (2H, m), 5.95-6.07 (1 H, m), 7.30 (1 H, s), 7.72 (1 H, d), 7.85 (1 H, d), 8.40-8.90 (2H, m), 9.30-9.60 (1 H, s).

### 9. 1-[7 β-({[2-t-butoxycarbonyl)amino-4-thiazolyl](methoxyimino)acetyl}amino)-2-(diphenylmethoxycarbonyl)-8-oxo-1-azabicyclo[4.2.0]oct-2-en-3-yl]-4-(dimethylamino)pyridinium trifluoromethanesulfonate, 81 % yield.

NMR (CD₃CN): 1.47 (9H, s), 2.67 (2H, m), 3.07 (6H, s), 3.97 (3H, s), 4.03-4.13 (1 H, m), 5.60-5.67 (1H, m), 6.58 (2H, d), 6.70 (1 H, s), 7.13-7.40 (10H, m), 7.62 (1 H, d), 7.78 (2H, d), 9.20 (1 H, s).

### 10. 1-[7 β-({[2-(triphenylmethyl)amino-4-thiazolyl](benzyloxyimino)acetyl}amino)-2-(diphenylmethoxycarbonyl)-8-oxo-1-azabicyclo[4.2.0]oct-2-en-3-yl]-3,4-dimethylpyridinium trifluoromethanesulfonate, 81 % yield.

NMR (CD₃CN): 1.62-1.83 (2H, m), 2.25 (3H, s), 2.40 (3H, s), 2.53-2.64 (2H, m), 3.96-4.07 (1H, m), 5.14 (2H, s), 5.45-5.58 (1 H, m), 6.60 (1 H, s), 6.73 (1 H, s), 7.10-7.60 (33H, m), 8.28-8.38 (2H, m).

### Example 11

### 1-(7 β-{[(2-amino-4-thiazolyl)(methoxyimino)acetyl]amino}-2-carboxy-8-oxo-1-azabicyclo[4.2.0]oct-2-en-3-yl)-pyridinium hydroxide, inner salt

To a solution of 22 mg of triphenylphosphine and 4.1 mg of palladium acetate in 10.5 ml of acetonitrile were added 300 mg of the diallyl protected intermediate of Example 1 F under a nitrogen atmosphere. After solution was complete, 2.0 ml of diethyl ether were added and the mixture cooled by means of an external ice bath. In dropwise fashion, 231 mcl of tributyl tin hydride were added. After 15 minutes, the ice bath was removed and the reaction mixture allowed to warm to room temperature. After 30 minutes, the ice bath was again applied and 0.88 ml of 1N hydrochloric acid added. After stirring for 10 minutes, the ice bath was removed and the reaction mixture stirred an additional 20 minutes. Diethyl ether and water were added and the layers were separated. The separated water layer was washed 3 times with diethyl ether/hexane. The water layer was then applied to a HP 20-SS column packed with water. Using a continuous gradient of 800 ml of water and 800 ml of 10% acetonitrile in water, the desired product was eluted and appropriate fractions combined and lyophilized providing 36 mg of the desired title product.
^{IR: 1768.}2 cm-1

NMR (DMSO, 500 MHz): 1.85-1.93 (1 H, m), 2.00-2.08 (1 H, m), 2.65-2.73 (1 H, m), 2.74-2.80 (1 H, m), 3.87 (3H, s), 3.88-3.93 (1 H, m), 5.43-5.42 (1H,m), 6.80 (1H,s), 7.23 (2H, s), 8.12 (2H, triplet), 8.60 (1H, triplet), 8.98 (2H, d), 9.39 (1 H, d).
MS: M⁺ + 1 = 443

### Examples 12-18

Employing the same procedure as taught above for Example 11 except that tetrahydrofuran was used in place of diethyl ether, the following compounds were prepared from the corresponding di-protected intermediates of Examples 2-8.

### 12. 6-(7 β-{[(2-amino-4-thiazolyl)(methoxyimino)acetyl]amino}-2-carboxy-8-oxo-1-azabicyclo[4.2.0]oct-2-en-3-yl)-2-(4-methoxyphenyl)thieno[2,3-c]pyridinium hydroxide, inner salt, 10% yield, m.p. = 210-211 °C.

MS: M⁺ + 1 = 605.

### 13. 3-(7 β-{[(2-amino-4-thiazolyl)(methoxyimino)acetyl]amino}-2-carboxy-8-oxo-1-azabicyclo[4.2.0]oct-2-en-3-yl)-1-methyl-1 H-imidazolium hydroxide, inner salt, 34% yield.

MS: M⁺ + 1 = 446
^{IR: 1765.}3 cm-1

NMR (DMSO): 1.70-2.00 (2H, m), 2.49-2.63 (2H, m), 3.77-3.87 (4H, m), 5.35-5.43 (1H,q), 6.77 (1H,s), 7.20 (1 H, s), 7.68 (1 H, s), 7.80 (1 H, s), 9.15 (1 H, s), 9.33 (1 H, d).

### 14. 5-(7-{[(2-amino-4-thiazolyl)(methoxyimino)acetyl]amino}-2-carboxy-8-oxo-1-azabicyclo[4.2.0]oct-2-en-3-yl)-3-methyl-3H-imidazol[4,5-c]pyridin-5-ium hydroxide, inner salt, 23% yield, m.p. = 219-220 °C.

MS: M⁺ + 1 = 497
^{IR: 1766.}5 cm-1

NMR (DMSO): 1.74-2.26 (2H, m), 2.74-2.90 (4H, m), 3.02 (1H, s), 5.32-5.50 (1H, m), 6.74 (1H, s), 7.14 (1 H, s), 8.16 (1 H, d), 8.50 (1 H, d), 8.54 (1 H, s), 9.32 (1 H, d), 9.66 (1 H, s).

### 15. 1-(7 β-{[(2-amino-4-thiazolyl)(methoxyimino)acetyl]amino}-2-carboxy-8-oxo-1-azabicyclo[4.2.0]oct-2-en-3-yl)-6,7-dihydro-5H-1-pyrindinium hydroxide, inner salt, 57% yield.

MS: M⁺ + 1 = 483
IR: 1765 cm⁻¹

NMR (DMSO): 1.80-2.27 (4H, m), 2.43-2.72 (2H, m), 3.07-3.40 (4H, m), 3.83 (3H, s), 3.87-4.00 (1H,m), 5.40-5.50 (1 H, m), 6.77 (1 H, s), 7.20 (2H, s), 7.77-7.87 (1 H, q), 8.37-8.43 (1 H, m), 8.55-8.73 (1 H, 2d), 9.30-9.40 (1 H, 2d).

### 16. 1-(7 β-{[(2-amino-4-thiazolyl)(methoxyimino)acetyl]amino}-2-carboxy-8-oxo-1-azabicyclo[4.2.0]oct-2-en-3-yl)-5,6,7,8-tetrahydroquinolinium hydroxide, inner salt, 56% yield, m.p. = 220-223 °C.

MS: M⁺ + 1 = 497
^{IR: 1769.}4 cm-1

### 17. 1-(7 β-{[(2-amino-4-thiazolyl)(methoxyimino)acetyl]amino}-2-carboxy-8-oxo-1-azabicyclo[4.2.0]oct-2-en-3-yl)-2,5-dimethylpyridinium hydroxide, inner salt, 31 % yield.

MS: M⁺ + 1 = 471

NMR (DMSO): 1.83-2.17 (2H, m), 2.40-2.43 (3H, 2s), 2.63-2.68 (3H, 2s), 2.73-2.80 (2H, m), 4.88 (3H, s), 4.03-4.20 (2H, m), 5.55-5.73 (1H,2 quartets), 6.80 (1H, s), 7.20 (2H, s), 7.80-7.97 (1H, m), 8.28-8.47 (1H, 2d), 8.70-8.80 (1H, 2s), 9.30-9.53 (1H, 2d).

### 18. 1-(7 β-{[(2-amino-4-thiazolyl)(methoxyimino)acetyl]amino }-2-carboxy-8-oxo-1-azabicyclo[ 4.2.0]oct-2-en-3-yl)-3,4-dimethylpyridinium hydroxide, inner salt, 47% yield.

MS: M⁺ + 1 = 471
IR: 1772.5 cₘ-¹

NMR (DMSO): 1.73-2.07 (2H, m), 2.35-2.77 (8H, m), 3.77-3.90 (4H, m), 5.38-5.43 (1 H, m), 6.77 (1 H, s), 7.20 (1 H, s), 7.87 (1 H, d), 8.63 (1 H, d), 8.77 (1 H, s), 9.38, (1 H, d).

### Example 19

### 1-(2-{[(4-nitrophenyl)methoxy]carbonyl}-8-oxo-7-[(phenoxyacetyl)amino]-1-azabicyclo[4.2.0]oct-2-en-3-yl)-pyridinium trifluoromethanesulfonate

The title product was prepared from p-nitrobenzyl 7,8-phenoxyacetylamino-3-trifluoromethanesul- fonyloxy-1-carba(dethia)-3-cephem-4-carboxylate in 94% yield according to the procedure of Example 1 F. MS.

### Example 20

### 1-(7 β-{[(2-amino-4-thiazolyl)(methoxyimino)acetyl]amino}-2-carboxy-8-oxo-1-azabicyclo[4.2.0]oct-2-en-3-yl)-4-(dimethylamino)pyridinium hydroxide, inner salt

To 175 mg of the intermediate of Example 9 was added a 0°C mixture of 1.2 ml of trifluoroacetic acid, 90 mcl of triethylsilane, and 1.2 ml of methylene chloride. After stirring at 0 _{°} C for 30 minutes, the external cooling bath was removed for five minutes and diethyl ether added to precipitate the product. The solid was recovered by centrifugation, washed three times with diethyl ether, and dried to provide 81 mg of crude title product. The material was dissolved in a small amount of water, filtered, and chromatographed by reverse phase HPLC employing 8% acetonitrile and 1% acetic acid in water as the eluant. Lyophilization of the desired fractions provided 41 mg of the desired title product.

NMR (DMSO): 1.70-2.00 (2H, m), 2.38-2.66 (2H, m), 3.20 (6H, s), 3.70-3.86 (2H, m), 5.30-5.37 (1 H, m), 6.73 (1 H, s), 6.95 (2H, d), 7.68 (2H, s), 8.10 (2H, d), 9.33 (1 H, d).

### Example 21

### 1-(7 β-{[(2-amino-4-thiazolyl)(benzyloxyimino)acetyl]amino}-2-carboxy-8-oxo-1-azabicyclo[4.2.0]oct-2-en-3-yl)-3,4-dimethylpyridinium hydroxide, inner salt

Fifty milligrams of the intermediate from Example 10 were added to 0.3 ml of trifluoroacetic acid and 9.7 ml of thiophenol under a nitrogen atmosphere and cooled by means of an external ethanol/ice bath. After stirring for 30 minutes at 0°C and 10 minutes with the ice bath removed, diethyl ether was added and the solid worked up in the same manner as in Example 21 above. Chromatography eluting with a continuous gradient of water to 12% acetonitrile in water and lyophilization provided 12 mg of the desired title product.

NMR (DMSO): 1.72-2.01 (2H, m), 2.45 (3H, s), 2.61 (3H, s), 2.72-2.88 (2H, m), 3.98-4.08 (1 H, m), 5.18 (2H, s), 5.64-5.76 (1 H, m), 6.84 (1 H, s), 7.24-7.36 (7H, m), 8.08 (1 H, d), 7.94 (1 H, d), 9.00 (1 H, s), 9.56 (1 H, d).

The 1-carbacephalosporins provided herein inhibit the growth of microorganisms pathogenic to man and animals. The compounds are broad spectrum antibiotics which are particularly effective against gram-positive bacteria.

This invention also provides a method for treating infectious diseases in man and animals and pharmaceutical formulations suitable for administration in the treatment method. The therapeutic method of this invention comprises administering to a man or animal an antibiotically effective non-toxic dose of a compound represented by the formula I wherein Rₐ is a negative charge, hydrogen, or a biologically labile group, or a solvate or pharmaceutically acceptable salt thereof.

An antibiotically effective amount is usually an amount between about 25 mg and about 2 grams. The compound, salt or ester may be administered in a single dose or in multiple doses throughout the day. Treatment may continue for a week to ten days or longer depending upon the duration of the infection. The particular dose and regimen can depend on such factors as the weight and age of the patient, the particular causative organism, the severity of the infection, the general health of the patient, and the tolerance of the individual to the antibiotic.

The 1-carbacephalosporins may be administered parenterally, orally, subcutaneously or rectally. As with other ,8-lactam antibiotics, the method of this invention may be used prophylactically to prevent infections after exposure or before possible exposure e.g., preoperatively. The antibiotic 1-carbacephalosporins may be administered by conventional methods e.g., in capsules, tablets, by syringe, or by intravenous drip.

The pharmaceutical formulations of the invention comprise an antibiotically effective non-toxic amount of a 1-carbacephalosporin represented by the formula I wherein Rₐ is a negative charge, hydrogen, or a biologically labile group, or a solvate or pharmaceutically acceptable salt thereof, and a pharmaceutical carrier.

Formulations for oral administration include capsules, tablets, lozenges, and liquid suspensions. The antibiotic or a salt or ester thereof in the form of a dry powder is encapsulated in gelatin capsules for oral use. The antibiotic may also be blended with an excipient, e.g., a stabilizer, prior to filling. Capsules may contain between about 100 mg and about 500 mg to provide unit dosage formulations.

Tablets containing between about 100 mg and 500 mg of the antibiotic or a salt or ester thereof are formulated by conventional means and may contain in addition a binding agent, disintegrating agent, stabilizing agent, antioxidant, etc.

Liquid preparations of the antibiotic may be prepared for infant and geriatric use. Pediatric suspensions are formulated with the antibiotic oral excipients such as suspending agents, flavoring agents, stabilizers, and the like. Solutions of the antibiotics likewise may be formulated with solubilizing agents, flavoring agents, sugar, water, etc.

Parenteral formulations of the antibiotics for injection are formulated with Water-for-Injection, Ringer's solution, physiological saline, or glucose solution. The antibiotic also may be administered in an intravenous fluid by the drip method.

For parenteral use, the antibiotic, a salt, solvate, or biologically labile ester thereof, is made up preferably in dry crystalline powder form or as a lyophilized powder and filled into vials. Such vials contain between about 100 mg and about 2 grams of antibiotic per vial.

## Claims (Claims for the following Contracting State(s) : AT, BE, CH, DE, FR, GB, IT, LI, NL, SE)

1. A compound of the Formula I 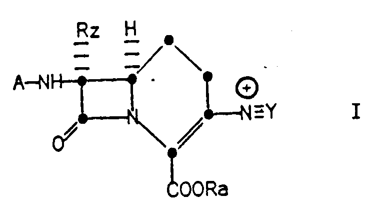
wherein A is hydrogen, an amino-protecting group, or an acyl group 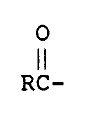
wherein R is hydrogen, C1-C6 alkyl, C1-C6 alkyl substituted by cyano, carboxy, halogen, amino, C₁-C₄ alkoxy, C₁-C₄ alkylthio, trifluoromethyl, or trifluoromethylthio, naphthyl, an optionally substituted phenyl group represented by the formula 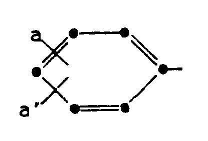
wherein a and a' independently are hydrogen, halogen, hydroxy, C₁-C₄ alkoxy, C₁-C₄ alkanoyloxy, C₁-C₄ alkyl, C₁-C₄ alkylthio, amino, C₁-C₄ alkanoylamino, C₁-C₄ alkylsulfonylamino, carboxy, carbamoyl, hydroxymethyl, aminomethyl, or carboxymethyl; a group represented by the formula 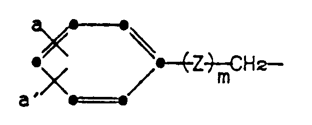
wherein a and a' have the same meanings as defined above, Z is O or S, and m is 0 or 1; a heteroarylmethyl group represented by the formula
Ri-CH₂-
wherein R₁ is thienyl, furyl, benzothienyl, benzofuryl, indolyl, triazolyl, tetrazolyl, oxazolyl, thiazolyl, oxadiazolyl, thiadiazolyl, and such heteroaryl groups substituted by amino, hydroxy, halogen, C₁-C₄ alkyl, C₁-C₄ alkoxy, C₁-C₄ alkylsulfonylamino; a substituted methyl group represented by the formula 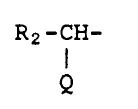
wherein R₂ is cyclohex-1,4-dienyl, or an optionally substituted phenyl group represented by the formula 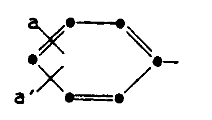
wherein a and a' have the above defined meanings, or R₂ is R₁ as defined above, and Q is hydroxy, C1-C4 alkanoyloxy, carboxy, sulfo, amino, or a substituted amino group represented by the formula 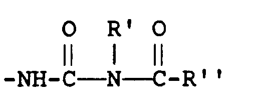
wherein R" is C₁-C₄ alkyl, furyl, thienyl, phenyl, halophenyl, nitrophenyl, styryl, halostyryl, nitrostyryl or a group 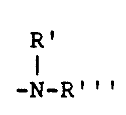
wherein each R' is independently hydrogen or C₁-C₃ alkyl, and R is hydrogen, C₁-C₃ alkylsulfonyl, C₁-C₃ alkyl, or C₁-C₄ alkanoyl; or Q is a substituted amino group represented by the formula 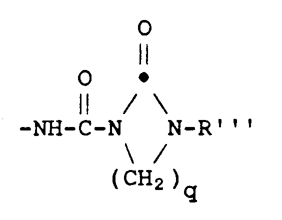
wherein R has the same meaning as defined above and q is 2 or 3; or Q is a substituted amino group represented by the formula 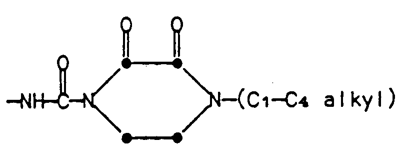
or a benzamido group represented by the formula 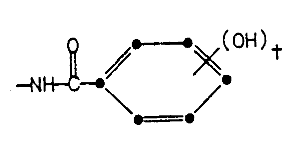 wherein t is 1 to 3;
a pyridone group 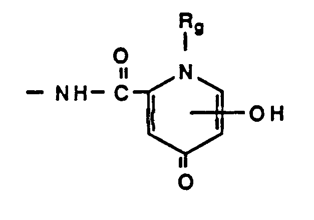
a pyridyl group 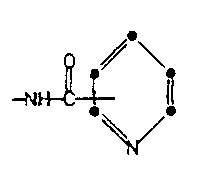
or said pyridyl group substituted by C₁-C₄ alkyl, amino, carboxy, hydroxy or halogen; an imidazolyl or pyrazolyl group 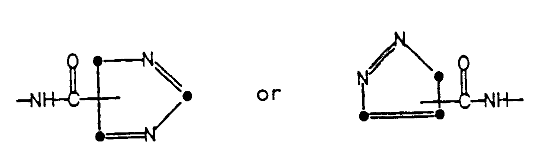 or said imidazolyl or pyrazolyl substituted by C₁-C₄ alkyl, carboxy, amino, or halogen;
a benzpyridazin-4-one group or tautomer thereof 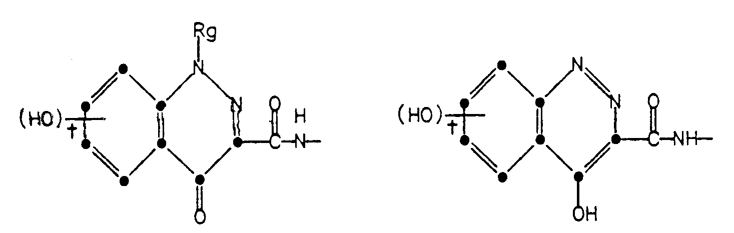 wherein Rg is hydrogen or C₁-C₃ alkyl, and t is 1 to 3;
or a benzpyranone group 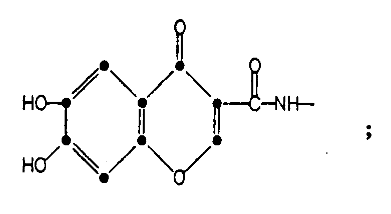
or R is a keto group or an oximino-substituted group represented by the formulae 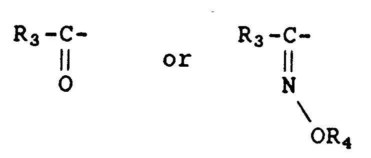
wherein R₃ is R₁ or R₂ as defined above and R₄ is hydrogen, C₁-C₄ alkyl, or a carboxy-substituted alkyl or cycloalkyl group represented by the formula 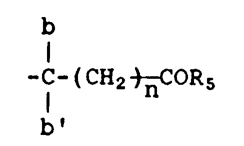
wherein b and b' independently are hydrogen or C₁-C₃ alkyl, or b and b', when taken together with the carbon to which they are bonded, form a 3- to 6-membered carbocyclic ring, n is 0-3, and R₅ is hydroxy, C₁-C₄ alkoxy, amino, C₁-C₄ alkylamino, or di(C₁-C₄ alkyl)amino; Rₐ is a negative charge, hydrogen, a biologically labile group, or a carboxy-protecting group;
R_{z} is hydrogen, C₁-C₄ alkoxy, C₁-C₄ alkylthio, or a formamido group, -NHCHO; -N≡Y is a quaternary ammonium group which is
a) a pyridinium ring, which may be substituted with one or two of the following substituents: C₁ to C₆ alkyl, C₁ to C₆ substituted alkyl, C₃ to C₇ cycloalkyl, C₃ to C₇ substituted cycloalkyl, C₅ to C₇ cycloalkenyl, C₅ to C₇ substituted cycloalkenyl, halo, cyano, hydroxy, protected hydroxy, C₁ to C₄ alkoxy, C₁ to C₄ alkythio, C₁ to C₄ alkylsulfoxide, C₁ to C₄ alkylsulfonyl, phenyl, substituted phenyl, formyl, C₂ to C₄ alkanoyl, benzyl, benzoyl, amino, protected amino, C₁ to C₄ alkylamino, di(Cᵢ to C₄ alkyl)amino, trifluoromethyl, carboxy, protected carboxy, C₁ to C₄ alkoxycarbonyl, aminomethyl, protected aminomethyl, carboxymethyl, protected carboxymethyl, carbamoyl, which may be substituted once or twice with a C₁ to C₆ alkyl group, an aminosulfonyl group (which may be substituted once or twice with a C₁ to C₆ alkyl group), a sulfonic acid, or a substituted or unsubstituted cyclic C₂ to C₁ o alkylene or heteroalkylene group;
b) a quinolinium, isoquinolinium, (1 or 2)-pyradizinium, (1 or 3)-pyrimidinium, N³-(C₁-C₄ alkyl)-imidazolinium, pyridazinium, phthalazinium, quinazolinium, purinium, pyrazinium, thiazolinium, isothiazolinium, oxazolinium, isoxasolinium, (3 or 4)-1,3,4-thiadiazolinium, (2 or 4)-1,2,4-thiadiazolinium, (2 or 5)-1,2,5-thiadiazolinium, (3 or 4)-1,3,4-oxadiazolinium, (2 or 4)-1,2,4-oxadiazolinium, or a (2 or 5)-1,2,5-oxadiazolinium ring, or the mono or di-substituted derivatives thereof, wherein the substituents can be the same or different (and in the case of the quinolinium or isoquinolinium rings, on one or both rings) and are amino, protected amino, C₁ to C₄ alkylamino, di-(C₁ to C₄ alkyl)amino, hydroxy, protected hydroxy, C₁ to C₄ alkoxy, C₁ to C₄ alkylthio, C₁ to C₄ alkylsulfoxide, C₁ to C₄ alkylsulfonyl, cyano, halo, C₁ to C₆ alkyl, C₁ to C₆ substituted alkyl, trifluoromethyl, C₃ to C₇ cycloalkyl, C₃ to C₇ substituted cycloalkyl, C₅ to C₇ cycloalkenyl, C₅ to C₇ substituted cycloalkenyl, sulfonic acid, carboxy, protected carboxy, C₁ to C₄ alkoxycarbonyl, hydroxy-(C₁ to C₃ alkyl), protected hydroxy-(C₁ to C₃ alkyl), formyl, C₂ to C₄ alkanoyl, an aminosulfonyl group (which may be substituted once or twice with a C₁ to C₆ alkyl group), carbamoyl (which may be substituted once or twice with a C₁ to C₆ alkyl group), aminomethyl, protected aminomethyl, carboxymethyl, (protected carboxy)methyl, phenyl, substituted phenyl, benzoyl or benzyl;
c) a group of the formula 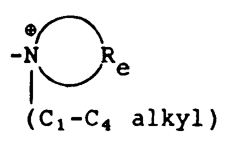 wherein Rₑ together with the nitrogen atom to which it is attached form a saturated or partially unsaturated 4 to 10 membered heterocyclic ring which may contain one or more further heteroatoms selected from oxygen, nitrogen or sulfur and wherein the substituent may be C₁ to C₆ alkyl, C₁ to C₆ substituted alkyl, C₃ to C₇ cycloalkyl, C₃ to C₇ substituted cycloalkyl, C₅ to C₇ cycloalkenyl, C₅ to C₇ substituted cycloalkenyl, halo, cyano, hydroxy, protected hydroxy, C₁ to C₄ alkoxy, C₁ to C₄ alkylthio, C₁ to C₄ alkylsulfoxide, C₁ to C₄ alkylsulfonyl, phenyl, substituted phenyl, formyl, C₂ to C₄ alkanoyl, benzyl, benzoyl, amino, protected amino, C₁ to C₄ alkylamino, di(Cᵢ to C₄ alkyl)amino, trifluoromethyl, carboxy, protected carboxy, C₁ to C₄ alkoxycarbonyl, aminomethyl, protected aminomethyl, carboxymethyl, protected carboxymethyl, carbamoyl (which may be substituted once or twice with a C₁ to C₆ alkyl group), aminosulfonyl (which may be substituted once or twice with a C₁ to C₆ alkyl group), or sulfonic acid, or the benzo-fused analogs of the substituted or unsubstituted, saturated or partially unsaturated ring, or
d) 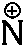 -N≡Y is tri(C₁-C₄ alkyl) ammonium;
or a solvate or a pharmaceutically acceptable non-toxic salt thereof.

2. A compound of claim 1 wherein Rₐ is hydrogen, a negative charge or a biologically labile group and A is an acyl group RCO.

3. A compound of claims 1 or 2 wherein R is
R₂-CH(NH₂)- or

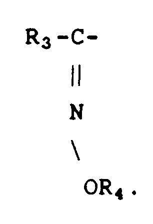

4. A compound of any one of claims 2 or 3 wherein -N=Y is substituted or unsubstituted pyridinium, quinolinium, isoquinolinium, 1- or 2-pyridazinium, 1- or 3-pyrimidinium, pyrazinium, thiazolinium, isothiazolinium, oxazolinium, isoxazolinium, 1,2,4-, 1,2,5-, or 1,3,4-thiadiazolinium or -oxadiazolinium, or a group of the formula

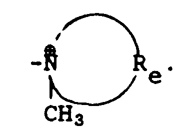

5. A compound of any one of claims 2 to 4 wherein RCO- is D-phenylglycyl, D-4-hydroxyphenylglycyl, D-2-thienylglycyl, D-benzothien-3-ylglycyl, (2-aminothiazol-4-yl)(methoxyimino)acetyl, or (2-aminooxazol-4-yl)-(methoxyimino)acetyl.

6. A pharmaceutical formulation which comprises a compound of Formula I as claimed in any one of claims 2 to 5, or a solvate or a pharmaceutically acceptable non-toxic salt thereof, and one or more pharmaceutically acceptable carriers, diluents or excipients therefor.

7. A compound of Formula I, as claimed in any one of the claims 2 to 5, or a solvate or a pharmaceutially acceptable non-toxic salt thereof, for use in treating bacterial infections in man and animals.

8. A process for preparing a compound of the Formula I as claimed in any one of claims 1 to 5, or a solvate or a pharmaceutically acceptable non-toxic salt thereof, which comprises
A) reacting a 3-triflate ester of the formula 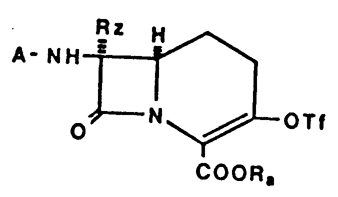 wherein Tf is a triflate moiety (-S0₂CF₃) and A and Rₐ are other than hydrogen with an amine N=Y and, optionally, deprotecting the resulting compound; or
B) esterifying a compound of the formula 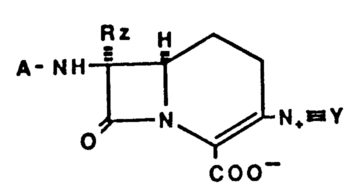 wherein A is other than hydrogen with a group Rₐ⁺; or
C) reacting a compound of the formula 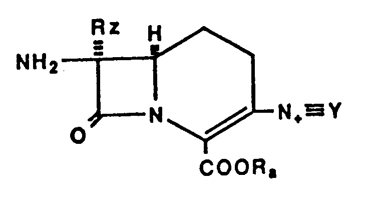 with a reagent AX wherein A is other than hydrogen and X is a good leaving group.

## Claims (Claims for the following Contracting State(s) : ES, GR)

1. A process for preparing a compound of the Formula I 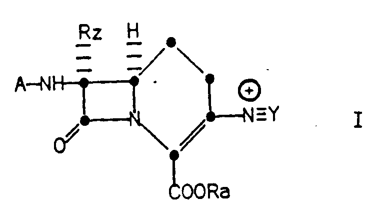
wherein A is hydrogen, an amino-protecting group, or an acyl group 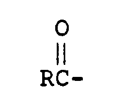
wherein R is hydrogen, C₁-C₆ alkyl, C₁-C₆ alkyl substituted by cyano, carboxy, halogen, amino, C₁-C₄ alkoxy, C₁-C₄ alkylthio, trifluoromethyl, or trifluoromethylthio, naphthyl, an optionally substituted phenyl group represented by the formula 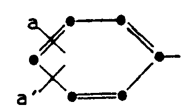
wherein a and a' independently are hydrogen, halogen, hydroxy, C₁-C₄ alkoxy, C₁-C₄ alkanoyloxy, C₁-C₄ alkyl, C₁-C₄ alkylthio, amino, C₁-C₄ alkanoylamino, C₁-C₄ alkylsulfonylamino, carboxy, carbamoyl, hydroxymethyl, aminomethyl, or carboxymethyl; a group represented by the formula 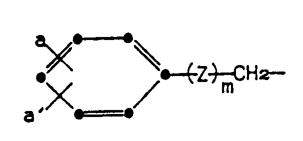
wherein a and a' have the same meanings as defined above, Z is O or S, and m is 0 or 1; a heteroarylmethyl group represented by the formula 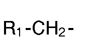
wherein R₁ is thienyl, furyl, benzothienyl, benzofuryl, indolyl, triazolyl, tetrazolyl, oxazolyl, thiazolyl, oxadiazolyl, thiadiazolyl, and such heteroaryl groups substituted by amino, hydroxy, halogen, C1-C4 alkyl, C1-C4 alkoxy, C₁-C₄ alkylsulfonylamino; a substituted methyl group represented by the formula 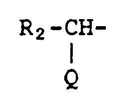
wherein R₂ is cyclohex-1,4-dienyl, or an optionally substituted phenyl group represented by the formula 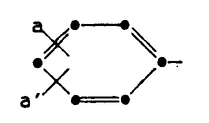
wherein a and a' have the above defined meanings, or R₂ is R₁ as defined above, and Q is hydroxy, C₁-C₄ alkanoyloxy, carboxy, sulfo, amino, or a substituted amino group represented by the formula 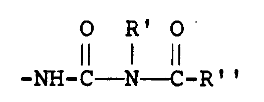
wherein R" is C₁-C₄ alkyl, furyl, thienyl, phenyl, halophenyl, nitrophenyl, styryl, halostyryl, nitrostyryl or a group 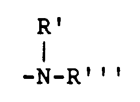
wherein each R' is independently hydrogen or C₁-C₃ alkyl, and R is hydrogen, C₁-C₃ alkylsulfonyl, C₁-C₃ alkyl, or C₁-C₄ alkanoyl; or Q is a substituted amino group represented by the formula 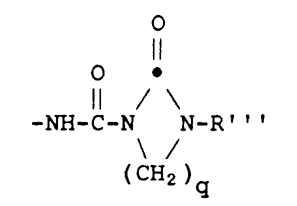
wherein R has the same meaning as defined above and q is 2 or 3;
or Q is a substituted amino group represented by the formula 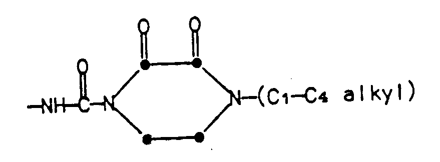
or a benzamido group represented by the formula 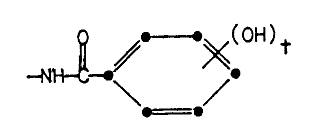 wherein t is 1 to 3;
a pyridone group 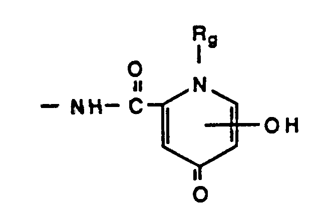
a pyridyl group 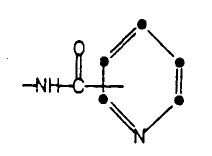
or said pyridyl group substituted by C₁-C₄ alkyl, amino, carboxy, hydroxy or halogen; an imidazolyl or pyrazolyl group 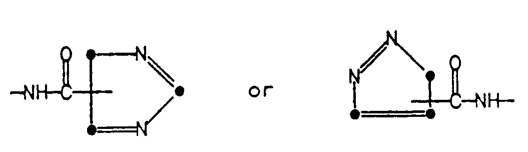 or said imidazolyl or pyrazolyl substituted by C₁-C₄ alkyl, carboxy, amino, or halogen;
a benzpyridazin-4-one group or tautomer thereof 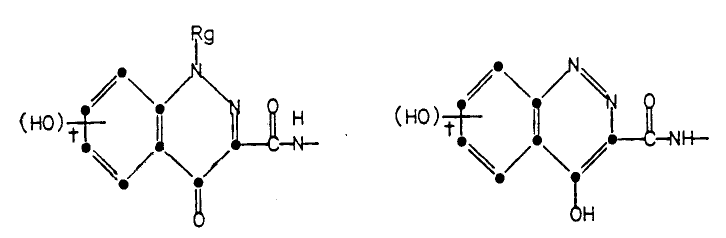 wherein Rg is hydrogen or C₁-C₃ alkyl, and t is 1 to 3;
or a benzpranone group 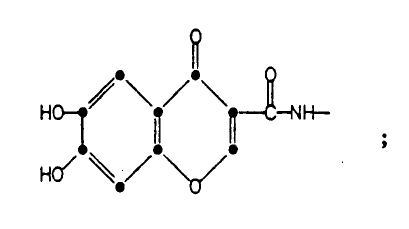
or R is a keto group or an oximino-substituted group represented by the formulae 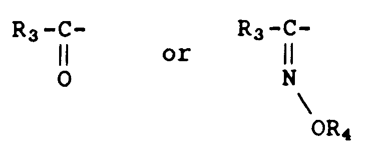
wherein R₃ is R₁ or R₂ as defined above and R₄ is hydrogen, C₁-C₄ alkyl, or a carboxy-substituted alkyl or cycloalkyl group represented by the formula 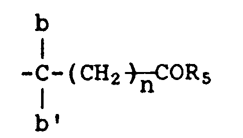
wherein b and b' independently are hydrogen or C1-C3 alkyl, or b and b', when taken together with the carbon to which they are bonded, form a 3- to 6-membered carbocyclic ring, n is 0-3, and R₅ is hydroxy, C₁-C₄ alkoxy, amino, C₁-C₄ alkylamino, or di(C₁-C₄ alkyl)amino; Rₐ is a negative charge, hydrogen, a biologically labile group, or a carboxy-protecting group;
R_{z} is hydrogen, C₁-C₄ alkoxy, C₁-C₄ alkylthio, or a formamido group, -NHCHO;
-N≡Y is a quaternary ammonium group which is
a) a pyridinium ring, which may be substituted with one or two of the following substituents: C₁ to C₆ alkyl, C₁ to C₆ substituted alkyl, C₃ to C₇ cycloalkyl, C₃ to C₇ substituted cycloalkyl, C₅ to C₇ cycloalkenyl, C₅ to C₇ substituted cycloalkenyl, halo, cyano, hydroxy, protected hydroxy, C₁ to C₄ alkoxy, C₁ to C₄ alkythio, C₁ to C₄ alkylsulfoxide, C₁ to C₄ alkylsulfonyl, phenyl, substituted phenyl, formyl, C₂ to C₄ alkanoyl, benzyl, benzoyl, amino, protected amino, C₁ to C₄ alkylamino, di(Cᵢ to C₄ alkyl)amino, trifluoromethyl, carboxy, protected carboxy, C₁ to C₄ alkoxycarbonyl, aminomethyl, protected aminomethyl, carboxymethyl, protected carboxymethyl, carbamoyl, which may be substituted once or twice with a C₁ to C₆ alkyl group, an aminosulfonyl group (which may be substituted once or twice with a C₁ to C₆ alkyl group), a sulfonic acid, or a substituted or unsubstituted cyclic C₂ to C₁ o alkylene or heteroalkylene group;
b) a quinolinium, isoquinolinium, (1 or 2)-pyradizinium, (1 or 3)-pyrimidinium, N³-(C₁-C₄ alkyl)-imidazolinium, pyridazinium, phthalazinium, quinazolinium, purinium, pyrazinium, thiazolinium, isothiazolinium, oxazolinium, isoxazolinium, (3 or 4)-1,3,4-thiadiazolinium, (2 or 4)-1,2,4-thiadiazolinium, (2 or 5)-1,2,5-thiadiazolinium, (3 or 4)-1,3,4-oxadiazolinium, (2 or 4)-1,2,4-oxadiazolinium, or a (2 or 5)-1,2,5-oxadiazolinium ring, or the mono or di-substituted derivatives thereof, wherein the substituents can be the same or different (and in the case of the quinolinium or isoquinolinium rings, on one or both rings) and are amino, protected amino, C₁ to C₄ alkylamino, di-(C₁ to C₄ alkyl)amino, hydroxy, protected hydroxy, C₁ to C₄ alkoxy, C₁ to C₄ alkylthio, C₁ to C₄ alkylsulfoxide, C₁ to C₄ alkylsulfonyl, cyano, halo, C₁ to C₆ alkyl, C₁ to C₆ substituted alkyl, trifluoromethyl, C₃ to C₇ cycloalkyl, C₃ to C₇ substituted cycloalkyl, C₅ to C₇ cycloalkenyl, C₅ to C₇ substituted cycloalkenyl, sulfonic acid, carboxy, protected carboxy, C₁ to C₄ alkoxycarbonyl, hydroxy-(C₁ to C₃ alkyl), protected hydroxy-(C₁ to C₃ alkyl), formyl, C₂ to C₄ alkanoyl, an aminosulfonyl group (which may be substituted once or twice with a C₁ to C₆ alkyl group), carbamoyl (which may be substituted once or twice with a C₁ to C₆ alkyl group), aminomethyl, protected aminomethyl, carboxymethyl, (protected carboxy)methyl, phenyl, substituted phenyl, benzoyl or benzyl;
c) a group of the formula 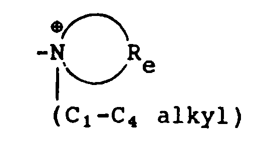 wherein Rₑ together with the nitrogen atom to which it is attached form a saturated or partially unsaturated 4 to 10 membered heterocyclic ring which may contain one or more further heteroatoms selected from oxygen, nitrogen or sulfur and wherein the substituent may be C₁ to C₆ alkyl, C₁ to C₆ substituted alkyl, C₃ to C₇ cycloalkyl, C₃ to C₇ substituted cycloalkyl, C₅ to C₇ cycloalkenyl, C₅ to C₇ substituted cycloalkenyl, halo, cyano, hydroxy, protected hydroxy, C₁ to C₄ alkoxy, C₁ to C₄ alkylthio, C₁ to C₄ alkylsulfoxide, C₁ to C₄ alkylsulfonyl, phenyl, substituted phenyl, formyl, C₂ to C₄ alkanoyl, benzyl, benzoyl, amino, protected amino, C₁ to C₄ alkylamino, di(Cᵢ to C₄ alkyl)amino, trifluoromethyl, carboxy, protected carboxy, C₁ to C₄ alkoxycarbonyl, aminomethyl, protected aminomethyl, carboxymethyl, protected carboxymethyl, carbamoyl (which may be substituted once or twice with a C₁ to C₆ alkyl group), aminosulfonyl (which may be substituted once or twice with a C₁ to C₆ alkyl group), or sulfonic acid, or the benzo-fused analogs of the substituted or unsubstituted, saturated or partially unsaturated ring, or
d) - 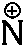 ≡Y is tri(C₁-C₄ alkyl) ammonium;
or a solvate or a pharmaceutically acceptable non-toxic salt thereof, which comprises
A) reacting a 3-triflate ester of the formula 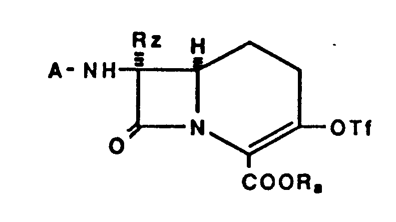 wherein Tf is a triflate moiety (-S0₂CF₃) and A and Rₐ are other than hydrogen with an amine N=Y and, optionally, deprotecting the resulting compound; or
B) esterifying a compound of the formula 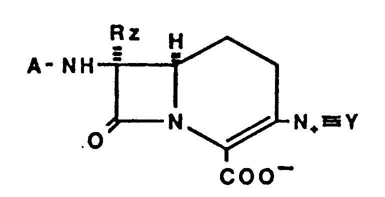 wherein A is other than hydrogen with a group Rₐ⁺; or
C) reacting a compound of the formula 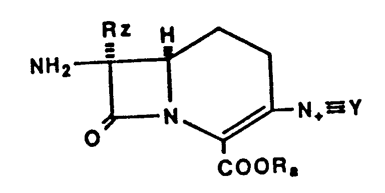 with a reagent AX wherein A is other than hydrogen and X is a good leaving group.

2. A process of claim 1 wherein Rₐ is hydrogen, a negative charge or a biologically labile group and A is an acyl group RCO.

3. A process of claim 2 wherein R is
R₂-CH(NH₂)- or

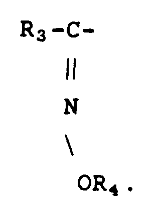

4. A process of either of claims 2 and 3 wherein - 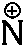 ≡Y is substituted or unsubstituted pyridinium, quinolinium, isoquinolinium, 1- or 2-pyridazinium, 1- or 3-pyrimidinium, pyrazinium, thiazolinium, isothiazolinium, oxazolinium, isoxazolinium, 1,2,4-, 1,2,5-, or 1,3,4-thiadiazolinium or -oxadiazolinium, or a group of the formula

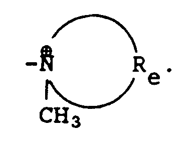

5. A process of any one of claims 2 to 4 wherein RCO- is D-phenylglycyl, D-4-hydroxyphenylglycyl, D-2-thienylglycyl, D-benzothien-3-ylglycyl, (2-aminothiazol-4-yl)(methoxyimino)acetyl, or (2-aminooxazol-4-yl)-(methoxyimino)acetyl.

6. A process for preparing a pharmaceutical formulation which comprises admixing a compound of Formula I, as defined in any one of claims 2 to 5, or a solvate or a pharmaceutically acceptable non-toxic salt thereof, with one or more pharmaceutically acceptable carriers, diluents or excipients therefor.

## Patentansprüche (Patentansprüche für folgende(n) Vertragsstaat(en) : AT, BE, CH, DE, FR, GB, IT, LI, NL, SE)

1. Verbindung der Formel I 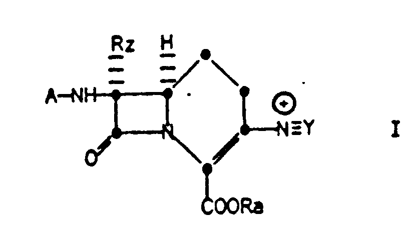
worin A Wasserstoff, eine Aminoschutzgruppe oder eine Acylgruppe 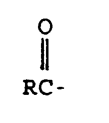
ist, worin R Wasserstoff, ein Cᵢ-C₆-Alkylrest, ein mit Cyano-, Carboxy-, Halogen-, Amino-, C1-C4-Alkoxy-, C₁-C₄-Alkylthio-, Trifluormethyl- oder Trifluormethylthioresten substituierter C₁-C₆-Alkylrest, Naphthylrest, eine gegebenenfalls substituierte Phenylgruppe der Formel 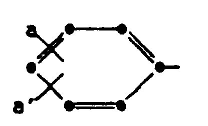
ist, worin a und a' unabhängig Wasserstoff, Halogen-, Hydroxy, C₁-C₄-Alkoxy-, C₁-C₄-Alkanoyloxy-, C₁-C₄-Alkyl-, C₁-C₄-Alkylthio-, Amino-, C1-C4-Alkanoylamino-, C₁-C₄-Alkylsulfonylamino-, Carboxy-, Carbamoyl-, Hydroxymethyl-, Aminomethyl- oder Carboxymethylreste sind; eine Gruppe der Formel 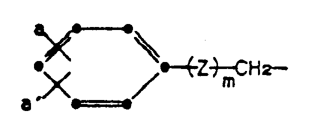
ist, worin a und a' dieselbe Bedeutung wie oben definiert haben, Z O oder S ist und m 0 oder 1 ist; eine Heteroarylmethylgruppe der Formel
R₁-CH₂-,
ist, worin R₁ eine Thienyl-, Furyl-, Benzothienyl-, Benzofuryl-, Indolyl-, Triazolyl-, Tetrazolyl-, Oxazolyl-, Thiazolyl-, Oxadiazolyl-, Thiadiazolylgruppe und eine derartige Heteroarylgruppe, die mit Amino-, Hydroxy-, Halogen-, C₁-C₄-Alkyl-, C₁-C₄-Alkoxy-, C1-C4-Alkylsulfonylaminoresten substituiert ist, ist; eine substituierte Methylgruppe der Formel 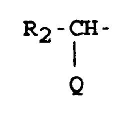
ist, worin R₂ ein Cyclohex-1,4-dienylrest oder eine gegebenenfalls substituierte Phenylgruppe der Formel 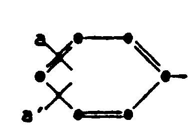
ist, worin a und a' die oben definierte Bedeutung haben oder R₂ wie oben für R₁ definiert ist und Q eine Hydroxy-, C1-C4-Alkanoyloxy-, Carboxy-, Sulfo-, Amino- oder substituierte Aminogruppe der Formel 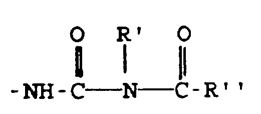
ist, worin R" eine C₁-C₄-Alkyl-, Furyl-, Thienyl-, Phenyl-, Halogenphenyl-, Nitrophenyl-, Styryl-, Halogenstyryl-, Nitrostyrylgruppe oder eine Gruppe 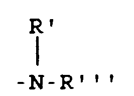
ist, worin jeder Rest R' unabhängig Wasserstoff oder ein C₁-C₃-Alkylrest ist und R"' Wasserstoff, ein C₁-C₃-Alkylsulfonyl-, C₁-C₃-Alkyl- oder C₁-C₄-Alkanoylrest ist; oder Q eine substituierte Aminogruppe der Formel 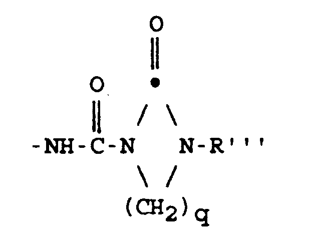
ist, worin R"' die gleiche Bedeutung wie oben definiert hat und q 2 oder 3 ist; oder Q eine substituierte Aminogruppe der Formel 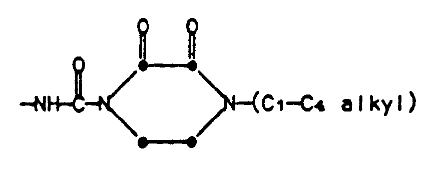
ist oder eine Benzamidogruppe der Formel 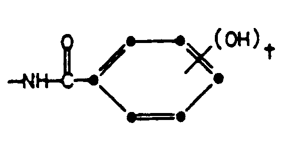
ist, worin t 1 bis 3 ist; eine Pyridongruppe 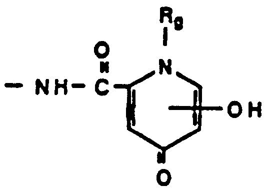
eine Pyridylgruppe 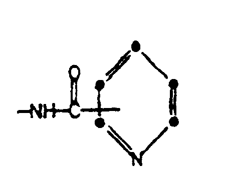
oder eine derartige Pyridylgruppe ist, die mit C₁-C₄-Alkyl-, Amino-, Carboxy-, Hydroxy- oder Halogenresten substituiert ist; eine Imidazolyl- oder Pyrazolylgruppe 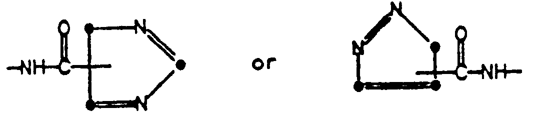
oder eine derartige Imidazolyl- oder Pyrazolylgruppe ist, die mit C₁-C₄-Alkyl-, Carboxy-, Amino- oder Halogenresten substituiert ist; eine Benzpyridazin-4-on-gruppe oder ein Tautomer davon 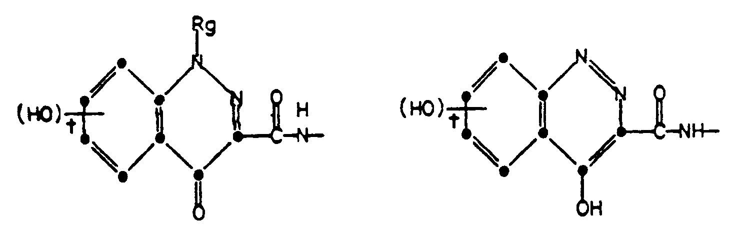
ist, worin Rg Wasserstoff oder ein C1-C3-Alkylrest ist und t 1 bis 3 ist; oder eine Benzpyranongruppe ist 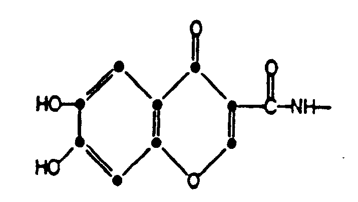
oder R eine Ketogrrnppe oder eine Oximino-substituierte Gruppe der Formeln 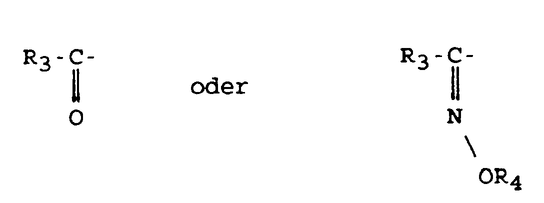
ist, worin R₃ R₁ oder R₂ wie oben definiert ist und R₄ Wasserstoff, ein C₁-C₄-Alkylrest oder eine mit Carboxy substituierte Alkyl- oder Cycloalkylgruppe der Formel 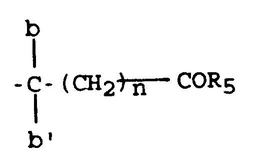
ist, worin b und b' unabhängig Wasserstoff oder C₁-C₃-Alkylreste sind oder b und b' zusammen mit dem Kohlenstoffatom, an das sie gebunden sind, einen 3- bis 6-gliedrigen carbocyclischen Ring bilden, n 0 bis 3 ist und R₅ ein Hydroxy-, C₁-C₄-Alkoxy-, Amino-, C₁-C₄-Alkylamino- oder Di(C₁-C₄-alkyl)-aminorest ist; Rₐ eine negative Ladung, Wasserstoff, eine biologisch labile Gruppe oder eine Carboxyschutzgruppe ist; R_{z} Wasserstoff, eine C₁-C₄-Alkoxy-, C₁-C₄-Alkylthio- oder Formamidogruppe -NHCHO ist; -N⁺≡Y eine quaternäre Ammoniumgruppe ist, die
a) ein Pyridiniumring ist, der mit einem oder zwei der folgenden Substituenten substituiert sein kann: C₁-C₆-Alkyl-, substituierten C₁-C₆-Alkyl-, C₃-C₇-Cycloalkyl-, substituierten C₃-C₇-Cycloalkyl-, C₅-C₇-Cycloalkenyl-, substituierten C₅-C₇-Cy-Cloalkenyl-,Halogen-, Cyano-, Hydroxy-, geschützten Hydroxy-, C₁-C₄-Alkoxy-, C₁-C₄-Alkylthio-, C₁-C₄-Alkylsulfoxid-, C₁-C₄-Alkylsulfonyl-, Phenyl-, substituierten Phenyl-, Formyl-, C₂-C₄-Alkanoyl-, Benzyl-, Benzoyl-, Amino-, geschützten Amino-, C1-C₄-Alkylamino-, Di(C₁-C₄-alkyl)amino-, Trifluormethyl-, Carboxy-, geschützten Carboxy-, C₁-C₄-Alkoxycarbonyl-, Aminomethyl-, geschützten Aminomethyl-, Carboxymethyl-, geschützten Carboxymethyl- , Carbamoylresten, die einfach oder zweifach mit C₁-C₆-Alkylgruppen substituiert sein können, einer Aminosulfonylgruppe (die einfach oder zweifach mit C₁-C₄-Alkylgruppen substituiert sein kann), einer sulfonsäure oder einer substituierten oder unsubstituierten cyclischen C₂- bis C₁₀-Alkylen-oder Heteroalkylengruppe;
b) ein Chinolinium-, Isochinolinium-, (1- oder 2-)-Pyradizinium-, (1- oder 3-)-Pyrimidinium-, N³⁻(C₁-C₄-Alkyl)imidazolinium-, Pyridazinium-, Phthalazinium-, Chinazolinium-, Purinium-, Pyrazinium-, Thiazolinium-, Isothiazolinium-, Oxazolinium-, Isoxazolinium-, (3- oder 4-)-1,3,4-Thiadiazolinium-, (2- oder 4-)-1,2,4-Thiadiazolinium-, (2- oder 5-)-1,2,5-Thiadiazolinium-, (3- oder 4-)-1,3,4-Oxadiazolinium-, (2-oder 4-)-1,2,4-Oxadiazolinium- oder ein (2- oder 5-)-1,2,5-Oxadiazoliniumring oder eines der mono-oder disubstituierten Derivate davon ist, worin die Substituenten gleich oder verschieden sein können (und im Fall der Chinolinium- oder Isochinoliniumringe an einem oder beiden Ringen sein können) und Amino-, geschützte Amino-, C₁-C₄-Alkylamino-, Di(C₁-C₄-alkyl)amino-, Hydroxy-, geschützte Hydroxy-, C₁-C₄-Alkoxy-, C₁-C₄-Alkylthio-, C1-C4-Alkylsulfoxid-, C₁-C₄-Alkylsulfonyl, Cyano-, Halogen-, C₁-C₆-Alkyl-, substituierte C₁-C₆-Alkyl-, Trifluormethyl-, C₃-C₇-Cycloalkyl-, substituierte C₃-C₇-Cycloalkyl-, C₅-C₇-Cycloalkenyl-, substituierte C₅-C₇-Cycloalkenyl-, Sulfonsäure-, Carboxy-, geschützte Carboxy-, C1-C4-Alkoxycarbonyl-, Hydroxy-(C₁-C₃-alkyl)-, geschützte Hydroxy-(C₁-C₃-alkyl)-, Formyl-, C₂-C₄-Alkanoylreste, eine Aminosulfonylgruppe (die einfach oder zweifach mit C₁-C₆-Alkylgruppen substituiert sein kann), Carbamoyl- (die einfach oder zweifach mit C₁-C₆-Alkylgruppen substituiert sein können), Aminomethyl-, geschützte Aminomethyl-, Carboxymethyl-, (geschützte Carboxy)methyl-, Phenyl-, substituierte Phenyl-, Benzoyl- oder Benzylreste sind;
c) eine Gruppe der Formel 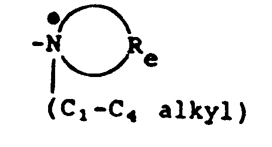 ist, worin Rₑ zusammen mit dem Stickstoffatom, an das es gebunden ist, einen gesättigten oder teilweise ungesättigten 4- bis 10-gliedrigen heterocyclischen Ring bildet, der ein oder mehrere weitere Heteroatome ausgewählt aus Sauerstoff, Stickstoff oder Schwefel enthalten kann und worin der Substituent ein C₁-C₆-Alkyl-, substituierter C₁-C₆-Alkyl-, C₃-C₇-Cycloalkyl-, substituierter C₃-C₇-Cycloalkyl-, C₅-C₇-Cycloalkenyl-, substituierter C₅-C₇-Cycloalkenyl-, Halogen-, Cyano-, Hydroxy-, geschützter Hydroxy-, C₁-C₄-Alkoxy-, C₁-C₄-Alkylthio, C₁-C₄-Alkylsulfoxid-, C₂-C₄-Alkanoyl-, Benzyl- , Benzoyl-, Amino-, geschützter Amino-, C₁-C₄-Alkylamino-, Di(C₁-C₄-alkyl)amino-, Trifluormethyl-, Carboxy-, geschützter Carboxy-, C₁-C₄-Alkoxycarbonyl-, Aminomethyl-, geschützter Aminomethyl-, Carboxymethyl-, geschützter Carboxymethyl-, Carbamoyl- (der einfach oder zweifach mit C₁-C₆-Alkylgruppen substituiert sein kann), Aminosulfonyl- (der einfach oder zweifach mit C₁-C₆-Alkylgruppen substituiert sein kann) oder Sulfonsäurerest ist oder eines der benzofusionierten Analoge des substituierten oder unsubstituierten, gesättigten oder teilweise ungesättigten Rings ist oder
d) -N⁺=Y ein Tri(C₁-C₄-alkyl)ammoniumrest ist; oder ein Solvat oder pharmazeutisch annehmbares nichttoxisches Salz davon.

2. Verbindung nach Anspruch 1, worin Rₐ Wasserstoff, eine negative Ladung oder eine biologisch labile Gruppe und A eine Acylgruppe RCO ist.

3. Verbindung nach Anspruch 1 oder 2, worin R R₂-CH(NH₂)- oder 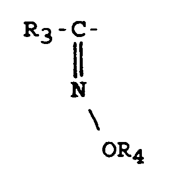 ist.

4. Verbindung nach einem der Ansprüche 2 oder 3, worin -N⁺=Y ein substituierter oder unsubstituierter Pyridinium-, Chinolinium-, Isochinolinium-, 1- oder 2-Pyridazinium-, 1- oder 3-Pyridinium-, Pyrazinium-, Thiazolinium-, Isothiazolinium-, Oxazolinium-, Isoxazolinium-, 1,2,4-, 1,2,5- oder 1,3,4-Thiadiazolinium-oder - oxadiazoliniumrest oder eine Gruppe der Formel 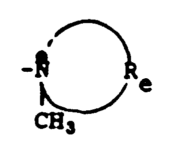 ist.

5. Verbindung nach einem der Ansprüche 2 bis 4, worin RCO- ein D-Phenylglycyl-, D-4-Hydroxyphenyl- glycyl-, D-2-Thienylglycyl-, D-Benzothien-3-ylglycyl-, (2-Aminothiazol-4-yl)(methoxyimino)acetyl- oder (2-Aminooxazol-4-yl)(methoxyimino)acetylrest ist.

6. Pharmazeutisches Präparat umfassend eine Verbindung der Formel I, nach einem der Ansprüche 2 bis 5 oder ein Solvat oder pharmazeutisch annehmbares nichttoxisches Salz davon und einen oder mehrere pharmazeutisch annehmbare Träger, Verdünnungsmittel oder Hilfsstoffe.

7. Verbindung der Formel I, nach einem der Ansprüche 2 bis 5 oder ein Solvat oder pharmazeutisch annehmbares nichttoxisches Salz davon zur Verwendung zur Behandlung von bakteriellen Infektionen bei Menschen und Tieren.

8. Verfahren zur Herstellung einer Verbindung der Formel 1 nach einem der Ansprüche 1 bis 5, oder eines Solvates oder eines pharmazeutisch annehmbaren nichttoxischen Salzes davon, umfassend, daß man
A) einen 3-Triflatester der Formel 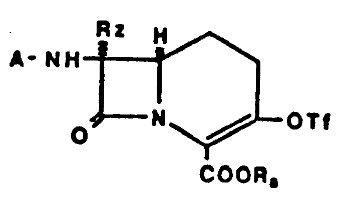 worin Tf ein Triflatrest (-S0₂CF₃) ist und A und Rₐ etwas anderes als Wasserstoff bedeuten, mit einem Amin N=Y umsetzt und gegebenenfalls die Schutzgruppen der entstehenden Verbindung abspaltet oder
B) eine Verbindung der Formel 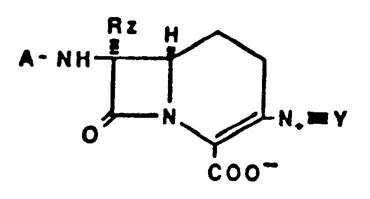 worin A etwas anderes als Wasserstoff bedeutet, mit einer Gruppe Rₐ⁺ verestert oder
C) eine Verbindung der Formel 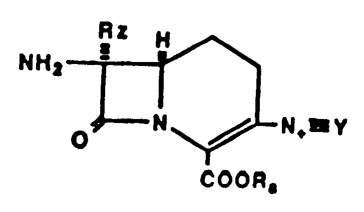 mit einem Reagenz AX, worin A etwas anderes als Wasserstoff bedeutet und X eine gute Abgangsgruppe ist, umsetzt.

## Patentansprüche (Patentansprüche für folgende(n) Vertragsstaat(en) : ES, GR)

1. Verfahren zur Herstellung einer Verbindung der Formel I 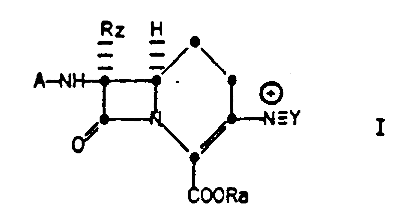
worin A Wasserstoff, eine Aminoschutzgruppe oder eine Acylgruppe 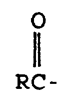
ist, worin R Wasserstoff, ein Cᵢ-C₆-Alkylrest, ein mit Cyano-, Carboxy-, Halogen-, Amino-, C₁-C₄-Alkoxy-, C₁-C₄-Alkylthio-, Trifluormethyl- oder Trifluormethylthioresten substituierter C₁-C₆-Alkylrest, Naphthylrest, eine gegebenenfalls substituierte Phenylgruppe der Formel 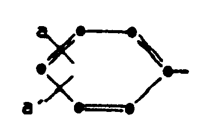
ist, worin a und a' unabhängig Wasserstoff, Halogen-, Hydroxy-, C₁-C₄-Alkoxy-, C₁-C₄-Alkanoyloxy-, C₁-C₄-Alkyl-, C₁-C₄-Alkylthio-, Amino-, Cᵢ-C₄-Alkanoylamino-, C1-C4-Alkylsulfonylamino-, Carboxy-, Carbamoyl-, Hydroxymethyl-, Aminomethyl- oder Carboxymethylreste sind; eine Gruppe der Formel 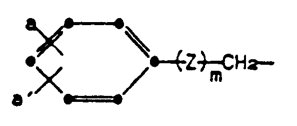
ist, worin a und a' dieselbe Bedeutung wie oben definiert haben, Z O oder S ist und m 0 oder 1 ist; eine Heteroarylmethylgruppe der Formel 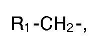
ist, worin R₁ eine Thienyl-, Furyl-, Benzothienyl-, Benzofuryl-, Indolyl-, Triazolyl-, Tetrazolyl-, Oxazolyl-, Thiazolyl-, Oxadiazolyl-, Thiadiazolylgruppe und eine derartige Heteroarylgruppe, die mit Amino-, Hydroxy-, Halogen-, C₁-C₄-Alkyl-, C₁-C₄-Alkoxy-, C₁-C₄-Alkylsulfonylaminoresten substituiert ist, ist; eine substituierte Methylgruppe der Formel 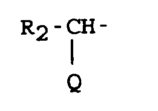
ist, worin R₂ ein Cyclohex-1,4-dienylrest oder eine gegebenenfalls substituierte Phenylgruppe der Formel 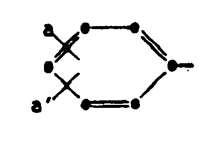
ist, worin a und a' die oben definierte Bedeutung haben oder R₂ wie oben für R₁ definiert ist und Q eine Hydroxy-, C₁-C₄-Alkanoyloxy-, Carboxy-, Sulfo-, Amino- oder substituierte Aminogruppe der Formel 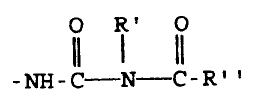
ist, worin R" eine C₁-C₄-Alkyl-, Furyl-, Thienyl-, Phenyl-, Halogenphenyl-, Nitrophenyl-, Styryl-, Halogenstyryl-, Nitrostyrylgruppe oder eine Gruppe 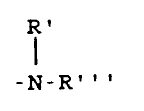
ist, worin jeder Rest R' unabhängig Wasserstoff oder ein C₁-C₃-Alkylrest ist und R"' Wasserstoff, ein C₁-C₃-Alkylsulfonyl-, C₁-C₃-Alkyl- oder C₁-C₄-Alkanoylrest ist; oder Q eine substituierte Aminogruppe der Formel 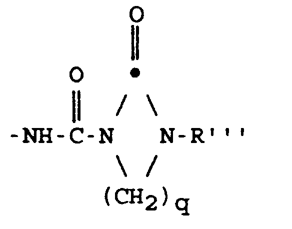
ist, worin R"' die gleiche Bedeutung wie oben definiert hat und q 2 oder 3 ist; oder Q eine substituierte Aminogruppe der Formel 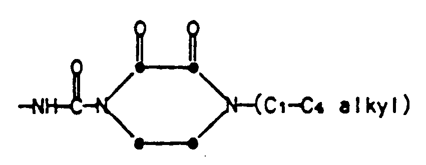
ist oder eine Benzamidogruppe der Formel 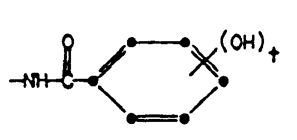
ist, worin t 1 bis 3 ist; eine Pyridongruppe 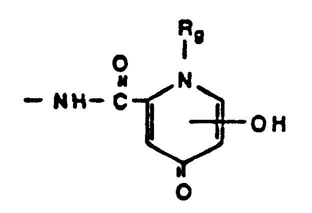 eine Pyridylgruppe 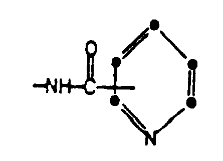
oder eine derartige Pyridylgruppe ist, die mit C₁-C₄-Alkyl-, Amino-, Carboxy-, Hydroxy- oder Halogenresten substituiert ist; eine Imidazolyl- oder Pyrazolylgruppe 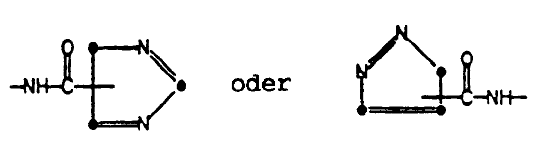
oder eine derartige Imidazolyl- oder Pyrazolylgruppe ist, die mit C₁-C₄-Alkyl-, Carboxy-, Amino- oder Halogenresten substituiert ist; eine Benzpyridazin-4-on-gruppe oder ein Tautomer davon 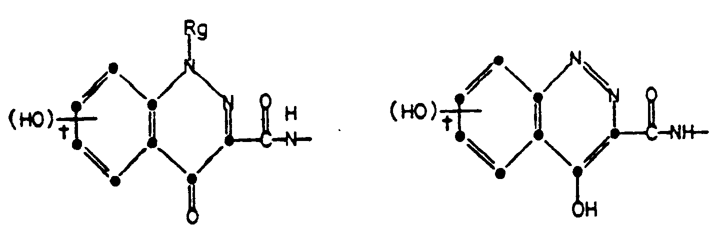
ist, worin Rg Wasserstoff oder ein C1-C3-Alkylrest ist und t 1 bis 3 ist; oder eine Benzpyranongruppe ist 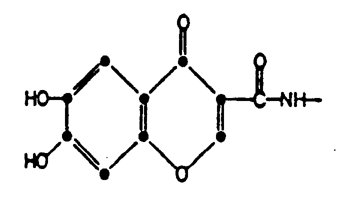
oder R eine Ketogruppe oder eine Oximino-substituierte Gruppe der Formeln 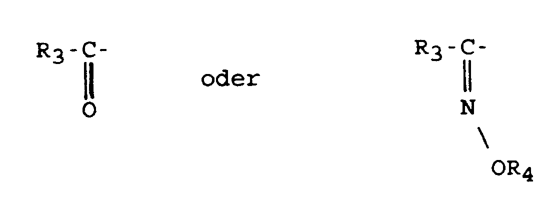
ist, worin R₃ R₁ oder R₂ wie oben definiert ist und R₄ Wasserstoff, ein C₁-C₄-Alkylrest oder eine mit
Carboxy substituierte Alkyl- oder Cycloalkylgruppe der Formel 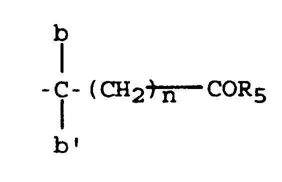
ist, worin b und b' unabhängig Wasserstoff oder C₁-C₃-Alkylreste sind oder b und b' zusammen mit dem Kohlenstoffatom, an das sie gebunden sind, einen 3- bis 6-gliedrigen carbocyclischen Ring bilden, n 0 bis 3 ist und R₅ ein Hydroxy-, C₁-C₄-Alkoxy-, Amino-, C₁-C₄-Alkylamino- oder Di(C₁-C₄-alkyl)-aminorest ist; Rₐ eine negative Ladung, Wasserstoff, eine biologisch labile Gruppe oder eine Carboxyschutzgruppe ist; R_{z} Wasserstoff, eine C₁-C₄-Alkoxy-, C₁-C₄-Alkylthio- oder Formamidogruppe -NHCHO ist; -N⁺≡Y eine quatenäre Ammoniumgruppe ist, die
a) ein Pyridiniumring ist, der mit einem oder zwei der folgenden Substituenten substituiert sein kann: C₁-C₆-Alkyl-, substituierten C₁-C₆-Alkyl-, C₃-C₇-Cycloalkyl-, substituierten C₃-C₇-Cycloalkyl-, C₅-C₇-Cycloalkenyl-, substituierten C₅-C₇-Cycloalkenyl-, Halogen-, Cyano-, Hydroxy-, geschützten Hydroxy-, C₁-C₄-Alkoxy-, C₁-C₄-Alkylthio-, C₁-C₄-Alkylsulfoxid-, C₁-C₄-Alkylsulfonyl-, Phenyl-, substituierten Phenyl-, Formyl-, C₂-C₄-Alkanoyl-, Benzyl-, Benzoyl-, Amino-, geschützten Amino-, C1-C₄-Alkylamino-, Di(C₁-C₄-alkyl)amino-, Trifluormethyl-, Carboxy-, geschützten Carboxy-, C₁-C₄-Alkoxycarbonyl-, Aminomethyl-, geschützten Aminomethyl-, Carboxymethyl-, geschützten Carboxymethyl- , Carbamoylresten, die einfach oder zweifach mit C₁-C₆-Alkylgruppen substituiert sein können, einer Aminosulfonylgruppe (die einfach oder zweifach mit Ci-C₆-Alkylgruppen substituiert sein kann), einer Sulfonsäure oder einer substituierten oder unsubstituierten cyclischen C₂- bis C₁₀-Alkylen-oder Heteroalkylengruppe;
b) ein Chinolinium-, Isochinolinium-, (1- oder 2-)-Pyradizinium-, (1- oder 3-)-Pyrimidinium-, N³⁻(C₁-C₄-Alkyl)imidazolinium-, Pyridazinium-, Phthalazinium-, Chinazolinium-, Purinium-, Pyrazinium-, Thiazolinium-, Isothiazolinium-, Oxazolinium-, Isoxazolinium-, (3- oder 4-)-1,3,4-Thiadiazolinium-, (2- oder 4-)-1,2,4-Thiadiazolinium-, (2- oder 5-)-1,2,5-Thiadiazolinium-, (3- oder 4-)-1,3,4-Oxadiazolinium-, (2-oder 4-)-1,2,4-Oxadiazolinium- oder ein (2- oder 5-)-1,2,5-Oxadiazoliniumring oder eines der mono-oder disubstituierten Derivate davon ist, worin die Substituenten gleich oder verschieden sein können (und im Fall der Chinolinium- oder Isochinoliniumringe an einem oder beiden Ringen sein können) und Amino-, geschützte Amino-, C₁-C₄-Alkylamino-, Di(C₁-C₄-alkyl)amino-, Hydroxy-, geschützte Hydroxy-, C₁-C₄-Alkoxy-, C₁-C₄-Alkylthio-, C₁-C₄-Alkylsulfoxid-, C₁-C₄-Alkylsulfonyl-, Cyano-, Halogen-, C₁-C₆-Alkyl-, substituierte C₁-C₆-Alkyl-, Trifluormethyl-, C₃-C₇-Cycloalkyl-, substituierte C₃-C₇-Cycloalkyl-, C₅-C₇-Cycloalkenyl-, substituierte C₅-C₇-Cycloalkenyl-, Sulfonsäure-, Carboxy-, geschützte Carboxy-, C1-C4-Alkoxycarbonyl-, Hydroxy-(C₁-C₃-alkyl)-, geschützte Hydroxy-(C₁-C₃-alkyl)-, Formyl-, C₂-C₄-Alkanoylreste, eine Aminosulfonylgruppe (die einfach oder zweifach mit C₁-C₆-Alkylgruppen substituiert sein kann), Carbamoyl- (die einfach oder zweifach mit C₁-C₆-Alkylgruppen substituiert sein können), Aminomethyl-, geschützte Aminomethyl-, Carboxymethyl-, (geschützte Carboxy)methyl-, Phenyl-, substituierte Phenyl-, Benzoyl- oder Benzylreste sind;
c) eine Gruppe der Formel 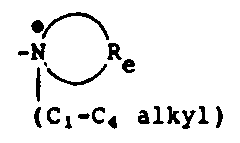 ist, worin Rₑ zusammen mit dem Stickstoffatom, an das es gebunden ist, einen gesättigten oder teilweise ungesättigten 4- bis 10-gliedrigen heterocyclischen Ring bildet, der ein oder mehrere weitere Heteroatome ausgewählt aus Sauerstoff, Stickstoff oder Schwefel enthalten kann und worin der Substituent ein C₁-C₆-Alkyl-, substituierter C₁-C₆-Alkyl-, C₃-C₇-Cycloalkyl-, substituierter C₃-C₇-Cycloalkyl-, C₅-C₇-Cycloalkenyl-, substituierter C₅-C₇-Cycloalkenyl-, Halogen-, Cyano-, Hydroxy-, geschützter Hydroxy-, C₁-C₄-Alkoxy-, C₁-C₄-Alkylthio-, C₁-C₄-Alkylsulfoxid-, C₁-C₄-Alkylsulfonyl-, Phenyl-, substituierter Phenyl-, Formyl-, C₂-C₄-Alkanoyl-, Benzyl-, Benzoyl-, Amino-, geschützter Amino-, C₁-C₄-Alkylamino-, Di(C₁-C₄-alkyl)amino-, Trifluormethyl-, Carboxy-, geschützter Carboxy-, C₁-C₄-Alkoxycarbonyl-, Aminomethyl-, geschützter Aminomethyl-, Carboxymethyl-, geschützter Carboxymethyl-, Carbamoyl- (der einfach oder zweifach mit C₁-C₆-Alkylgruppen substituiert sein kann), Aminosulfonyl- (der einfach oder zweifach mit Cᵢ-C₆-Alkylgruppen substituiert sein kann) oder Sulfonsäurerest ist oder eines der benzokondensierten Analoge des substituierten oder unsubstituierten, gesättigten oder teilweise ungesättigten Rings ist oder
d) -N⁺=Y ein Tri(C₁-C₄-alkyl)ammoniumrest ist; oder eines Solvats oder pharmazeutisch annehmbaren nichttoxischen Salzes davon umfassend, daß man
A) einen 3-Triflatester der Formel 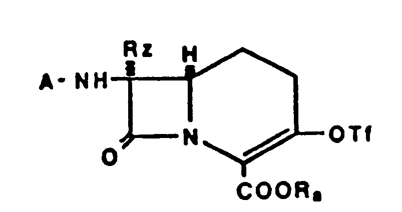 worin Tf ein Triflatrest (-S0₂CF₃) ist und A und Rₐ etwas anderes als Wasserstoff bedeuten, mit einem Amin N=Y umsetzt und gegebenenfalls die Schutzgruppen der entstehenden Verbindung abspaltet oder
B) eine Verbindung der Formel 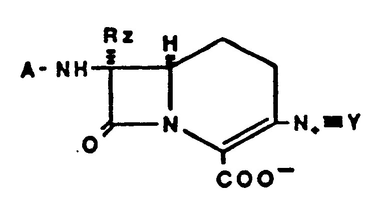 worin A etwas anderes als Wasserstoff bedeutet, mit einer Gruppe Rₐ⁺ verestert oder
C) eine Verbindung der Formel 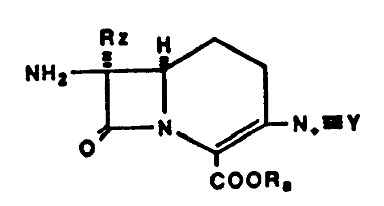 mit einem Reagenz AX, worin A etwas anderes als Wasserstoff bedeutet und X eine gute Abgangsgruppe ist, umsetzt.

2. Verfahren nach Anspruch 1, worin Rₐ Wasserstoff, eine negative Ladung oder eine biologisch labile Gruppe ist und A eine Acylgruppe RCO ist.

3. Verfahren nach Anspruch 2, worin R R₂-CH(NH₂)- oder 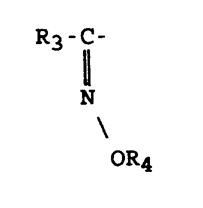 ist.

4. Verfahren nach einem der Ansprüche 2 oder 3, worin -N⁺=Y ein substituierter oder unsubstituierter Pyridinium-, Chinolinium-, Isochinolinium-, 1- oder 2-Pyridazinium-, 1- oder 3-Pyridinium-, Pyrazinium-, Thiazolinium-, Isothiazolinium-, Oxazolinium-, Isoxazolinium-, 1,2,4-, 1,2,5- oder 1,3,4-Thiadiazolinium-oder - oxadiazoliniumrest oder eine Gruppe der Formel 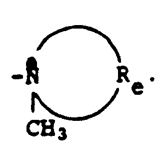 ist.

5. Verfahren nach einem der Ansprüche 2 bis 4, worin RCO- ein D-Phenylglycyl-, D-4-Hydroxyphenylgly- cyl-, D-2-Thienylglycyl-, D-Benzothien-3-ylglycyl-, (2-Aminothiazol-4-yl) (methoxyimino)acetyl oder (2-Aminooxazol-4-yl) (methoxyimino)acetylrest ist.

6. Verfahren zur Herstellung eines pharmazeutischen Präparats umfassend, daß man eine Verbindung der Formel I, wie in einem der Ansprüche 2 bis 5 definiert, oder ein Solvat oder pharmazeutisch annehmbares nichttoxisches Salz davon mit einem oder mehreren pharmazeutisch annehmbaren Trägern, Verdünnungsmitteln oder Hilfsstoffen vermischt.

## Revendications (Revendications pour l'(les) Etat(s) contractant(s) suivant(s) : suivants : AT, BE, CH, DE, FR, GB, IT, LI, NL, SE)

1. Composé de Formule 1 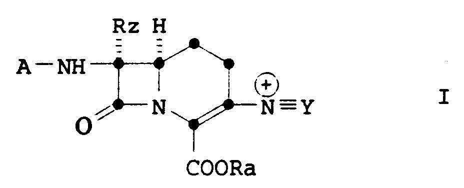
dans laquelle A est un atome d'hydrogène, un groupe amino-protecteur ou un groupe acyle 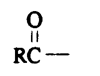
dans lequel R est un atome d'hydrogène, un groupe alkyle en Ci à C₆, alkyle en Ci à C₆ substitué par un groupe cyano, carboxyle, un atome d'halogène, un groupe amino, alcoxy en Ci à C₄, alkylthio en C₁ à C₄, trifluorométhyle ou trifluorométhylthio, naphtyle, un groupe phényle facultativement substitué, représenté par la formule 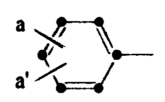
dans laquelle a et a' sont, indépendamment l'un de l'autre, un atome d'hydrogène, d'halogène, un groupe hydroxyle, alcoxy en Ci à C₄, alcanoyloxy en Ci à C₄, alkyle en Ci à C₄, alkylthio en Ci à C₄, amino, alcanoylamino en Ci à C₄, alkylsulfonylamino en Ci à C₄, carboxyle, carbamoyle, hydroxymé- thyle, aminométhyle ou carboxyméthyle; un groupe représenté par la formule 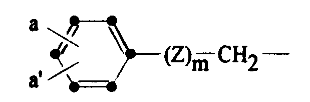
dans laquelle a et a' ont les mêmes significations que celles définies ci-dessus, Z représente O ou S, et vaut 0 ou 1;
un groupe hétéroarylméthyle représenté par la formule
R₁-CH₂-
dans laquelle R₁ est un groupe thiényle, furyle, benzothiényle, benzofuryle, indolyle, triazolyle, tétrazolyle, oxazolyle, thiazolyle, oxadiazolyle, thiadiazolyle et de tels groupes hétéroaryles substitués par un groupe amino, hydroxyle, un atome d'halogène, un groupe alkyle en Ci à C₄, alcoxy en C₁ à C₄, alkylsulfonylamino en C₁ à C₄; un groupe méthyle substitué représenté par la formule 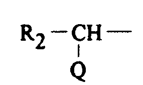
dans laquelle R₂ est un groupe cyclohex-1,4-diényle ou un groupe phényle facultativement substitué représenté par la formule 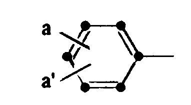
dans laquelle a et a' ont les significations définies ci-dessus, ou R₂ vaut R₁ tel que défini ci-dessus, et Q est un groupe hydroxyle, alcanoyloxy en C₁ à C₄, carboxyle, sulfo, amino ou un groupe amino substitué représenté par la formule 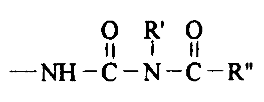
dans laquelle R" représente un groupe alkyle en C₁ à C₄, furyle, thiényle, phényle, halophényle, nitrophényle, styryle, halostyryle, nitrostyryle ou un groupe 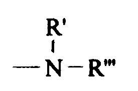
dans lequel chaque R' représente, indépendamment l'un de l'autre, un atome d'hydrogène ou un groupe alkyle en Ci à C₃, et R"' est un atome d'hydrogène, un groupe alkylsulfonyle en Ci à C₃, alkyle en C₁ à C₃ ou alcanoyle en C₁ à C₄;
ou Q est un groupe amino substitué représenté par la formule 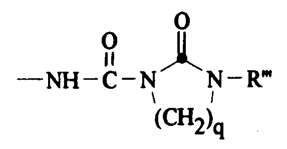 dans laquelle R''' a la même signification que celle définie ci-dessus et q vaut 2 ou 3;
ou Q est un groupe amino substitué représenté par la formule 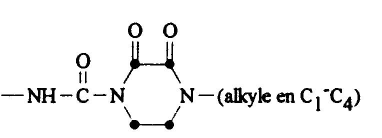
ou un groupe benzmido représenté par la formule 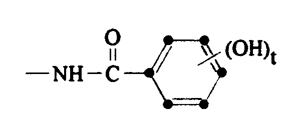 dans laquelle t vaut 1 à 3;
un groupe pyridone 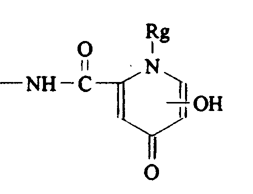
un groupe pyridyle 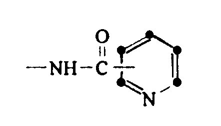
ou ledit groupe pyridyle substitué par un groupe alkyle en Ci à C₄, amino, carboxyle, hydroxyle ou un atome d'halogène; un groupe imidazolyle ou pyrazolyle 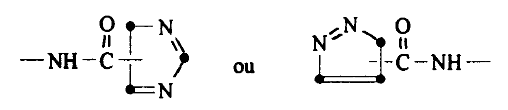
ou ledit groupe imidazolyle ou pyrazolyle substitué par un groupe alkyle en Ci à C₄, carboxyle, amino ou un atome d'halogène;
un groupe benzpyridazin-4-one ou un tautomère de celui-ci 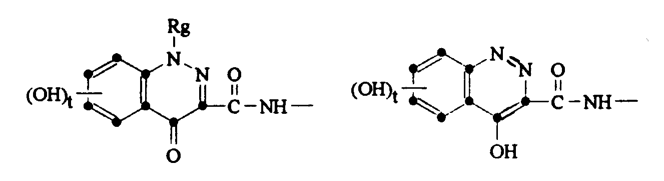 dans lequel Rg est un atome d'hydrogène ou un groupe alkyle en Ci à C₃, et t vaut 1 à 3;
ou un groupe benzpyranone 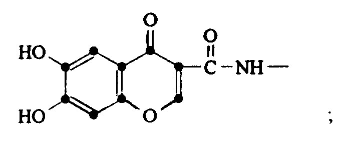 ou R est un groupe céto ou un groupe oximino-substitué représenté par les formules 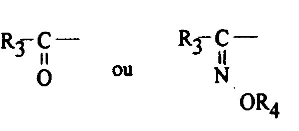
dans lesquelles R₃ vaut R₁ ou R₂ tels que définis ci-dessus et R₄ représente un atome d'hydrogène, un groupe alkyle en Ci à C₄ ou un groupe alkyle ou cycloalkyle carboxy-substitué représenté par la formule 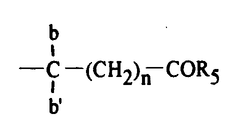
dans laquelle b et b' représentent, indépendamment l'un de l'autre, un atome d'hydrogène ou un alkyle en Ci à C₃, ou b et b', lorsqu'ils sont pris ensemble avec le carbone auquel ils sont attachés, forment un noyau carbocyclique de trois à six membres, n vaut 0 à 3 et R₅ est un groupe hydroxyle, alcoxy en Ci à C₄, amino, alkylamino en C₁ à C₄ ou un groupe di(alkyle en C₁ à C₄)amino; Rₐ représente une charge négative, un atome d'hydrogène, un groupe biologiquement instable ou un groupe carboxy-protecteur; R_{z} représente un atome d'hydrogène, un groupe alcoxy en C₁ à C₄, alkylthio en C₁ à C₄ ou un groupe formamido, -NHCHO;
- 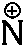 ≡Y représente un groupe ammonium quaternaire qui est
a. un noyau pyridinium, qui peut être substitué par un ou deux des substituants suivants : alkyle en C₁ à C₆, alkyle en C₁ à C₆ substitué, cycloalkyle en C₃ à C₇, cycloalkyle en C₃ à C₇ substitué, cyclalcényle en C₅ à C₇, cyclalcényle en C₅ à C₇ substitué, halogène, cyano, hydroxyle, hydroxyle protégé, alcoxy en Ci à C₄, alkylthio en Ci à C₄, alkylsulfoxyde en Ci à C₄, alkylsulfonyle en Ci à C₄, phényle, phényle substitué, formyle, alcanoyle en C₂ à C₄, benzyle, benzoyle, amino, amino protégé, alkylamino en Ci à C₄, di(alkyle en Ci à C₄)amino, trifluorométhyle, carboxyle, carboxyle protégé, alcoxycarbonyle en Ci à C₄, aminométhyle, aminométhyle protégé, carboxyméthyle, carboxyméthyle protégé, carbamoyle, qui peut être substitué une ou deux fois par un groupe alkyle en Ci à C₆, un groupe aminosulfonyle (qui peut être substitué une ou deux fois par un groupe alkyle en Ci à C₆), un acide sulfonique ou un groupe alkylène ou hétéroalkylène cyclique en C₂ à C₁₀ substitué ou non-substitué;
b. un noyau quinolinium, isoquinolinium, (1 ou 2)-pyradizinium, (1 ou 3)-pyrimidinium, N³⁻(alkyle en C₁ à C₄)imidazolinium, pyridazinium, phthalazinium, quinazolinium, purinium, pyrazinium, thiazolinium, isothiazolinium, oxazolinium, isoxazolinium, (3 ou 4)-1,3,4-thiadiazolinium, (2 ou 4)-1,2,4-thiadiazolinium, (2 ou 5)-1,2,5-thiadiazolinium, (3 ou 4)-1,3,4-oxadiazolinium, (2 ou 4)-1,2,4-oxadiazolinium ou (2 ou 5)-1,2,5-oxadiazolinium, ou leurs dérivés mono- ou disubstitués, dans lesquels les substituants peuvent être identiques ou différents (et dans le cas des noyaux quinolinium ou isoquinolinium, sur un ou sur les deux noyaux) et représentent un groupe amino, amino protégé, alkylamino en Ci à C₄, di(alkyle en Ci à C₄)amino, hydroxyle, hydroxyle protégé, alcoxy en Ci à C₄, alkylthio en Ci à C₄, alkylsulfoxyde en Ci à C₄, alkylsulfonyle en Ci à C₄, cyano, un atome d'halogène, un groupe alkyle en Ci à C₆, alkyle en C₁ à C₆ substitué, trifluorométhyle, cycloalkyle en C₃ à C₇, cycloalkyle en C₃ à C₇ substitué, cyclalcényle en C₅ à C₇, cyclalcényle en C₅ à C₇ substitué, un acide sulfonique, un groupe carboxyle, carboxyle protégé, alcoxycarbonyle en C₁ à C₄, hydroxyalkyle en C₁ à C₃, hydroxyalkyle en C₁ à C₃ protégé, formyle, alcanoyle en C₂ à C₄, un groupe aminosulfonyle (qui peut être substitué une ou deux fois par un groupe alkyle en C₁ à C₆), carbamoyle (qui peut être substitué une ou deux fois par un groupe alkyle en C₁ à C₆), aminométhyle, aminométhyle protégé, carboxyméthyle, (carboxy protégé)méthyle, phényle, phényle substitué, benzoyle ou benzyle;
c. un groupe de formule 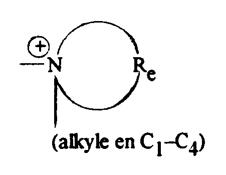 dans laquelle Rₑ, pris ensemble avec l'atome d'azote auquel il est lié, forment un noyau hétérocyclique de quatre à dix membres saturé ou partiellement insaturé, qui peut contenir un ou plusieurs hétéroatomes supplémentaires choisis parmi les atomes d'oxygène, d'azote ou de soufre et dans lequel le substituant peut être un groupe alkyle en C₁ à C₆, alkyle en C₁ à C₆ substitué, cycloalkyle en C₃ à C₇, cycloalkyle en C₃ à C₇ substitué, cyclalcényle en C₅ à C₇, cyclalcényle en C₅ à C₇ substitué, un atome d'halogène, un groupe cyano, hydroxyle, hydroxyle protégé, alcoxy en C₁ X C₄, alkylthio en C₁ à C₄, alkylsulfoxyde en C₁ à C₄, alkylsulfonyle en C₁ à C₄, phényle, phényle substitué, formyle, alcanoyle en C₂ à C₄, benzyle, benzoyle, amino, amino protégé, alkylamino en C₁ à C₄, di(alkyle en C₁ à C₄)amino, trifiuorométhyle, carboxyle, carboxyle protégé, alcoxycarbonyle en C₁ à C₄, aminométhyle, aminométhyle protégé, carboxyméthyle, carboryméthyle protégé, carbamoyle (qui peut être substitué une ou deux fois par un groupe alkyle en C₁ à C₆), aminosulfonyle (qui peut être substitué une ou deux fois par un groupe alkyle en C₁ à C₆) ou un acide sulfonique, ou les analogues benzo-fusionnés du noyau saturé ou partiellement insaturé substitué ou non-substitué, ou
d. - 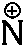 ≡Y représente un groupe ammonium tri(alkyle en C₁ à C₄); ou un solvate ou un sel non-toxique et pharmaceutiquement acceptable de ceux-ci.

2. Composé selon la revendication 1 dans lequel Rₐ est un atome d'hydrogène, une charge négative ou un groupe biologiquement instable et A est un groupe acyle RCO-.

3. Composé selon les revendications 1 ou 2 dans lequel R représente
R₂-CH(NH₂)- ou

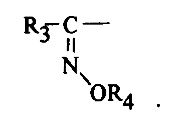

4. Composé selon n'importe laquelle des revendications 2 ou 3 dans lequel - 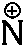 ≡Y représente un noyau, substitué ou non-substitué, de pyridinium, quinolinium, isoquinolinium, 1- ou 2-pyridazinium, 1- ou 3- pyrimidinium, pyrazinium, thiazolinium, isothiazolinium, oxazolinium, isoxazolinium, 1,2,4-, 1,2,5- ou 1,3,4-thiadiazolinium ou -oxadiazolinium, ou un groupe de formule

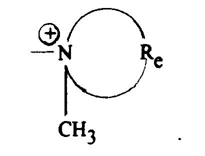

5. Composé selon n'importe laquelle des revendications 2 à 4, dans lequel RCO-représente un groupe D-phénylglycyle, D-4-hydroxyphénylglycyle, D-2-thiénylglycyle, D-benzothièn-3-ylglycyle, (2-aminothiazol-4-yl)(méthoxyimino)acétyle ou (2-aminooxazol-4-yl)(méthoxyimino)acétyle.

6. Formulation pharmaceutique qui comprend un composé de Formule I telle que revendiqué dans n'importe laquelle des revendications 2 à 5, ou un de ses solvates ou sels non-toxiques et pharmaceutiquement acceptables, et un ou plusieurs supports, diluants ou excipients pharmaceutiquement acceptables pour celui-ci.

7. Composé de Formule I, tel que revendiqué dans n'importe laquelle des revendications 2 à 5, ou un de ses solvates ou sels non-toxiques et pharmaceutiquement acceptables, destiné à l'utilisation pour le traitement d'infections bactériennes chez l'homme et les animaux.

8. Procédé pour la préparation d'un composé de Formule I, tel què revendiqué dans n'importe laquelle des revendications 1 à 5 ou un de ses solvates ou sels non-toxiques ou pharmaceutiquement acceptables, qui comprend
a. la mise en réaction d'un ester 3-triflate de formule 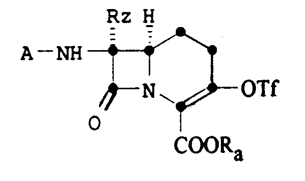 dans laquelle Tf est une fraction triflate (-S0₂CF₃) et A et Rₐ sont différents d'un atome d'hydrogène avec une amine N=Y et, facultativement la déprotection du composé résultant; ou
b. l'estérification d'un composé de formule 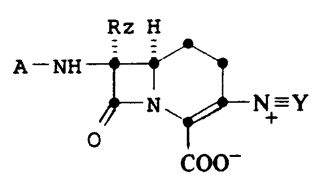 dans laquelle A est différent d'un atome d'hydrogène avec un groupe Rₐ⁺; ou c. la mise en réaction d'un composé de formule 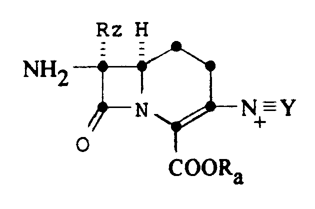 avec un réactif AX dans lequel A est différent d'un atome d'hydrogène et X est un bon groupe sortant.

## Revendications (Revendications pour l'(les) Etat(s) contractant(s) suivant(s) : suivants : ES, GR)

1. Procédé pour la préparation d'un composé de Formule I 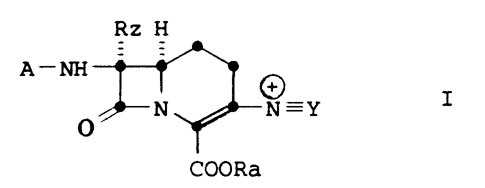
dans laquelle A est un atome d'hydrogène, un groupe amino-protecteur ou un groupe acyle 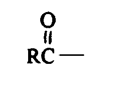
dans lequel R est un atome d'hydrogène, un groupe alkyle en Ci à C₆, alkyle en Ci à C₆ substitué par un groupe cyano, carboxyle, un atome d'halogène, un groupe amino, alcoxy en Ci à C₄, alkylthio en C₁ à C₄, trifluorométhyle ou trifluorométhylthio, naphtyle, un groupe phényle facultativement substitué, représenté par la formule 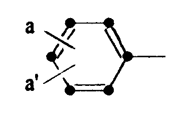
dans laquelle a et a' sont, indépendamment l'un de l'autre, un atome d'hydrogène, d'halogène, un groupe hydroxyle, alcoxy en Ci à C₄, àlcanoyloxy en Ci à C₄, alkyle en C₁ à C₄, alkylthio en C₁ à C₄, amino, alcanoylamino en C₁ à C₄, alkylsulfonylamino en C₁ à C₄, carboxyle, carbamoyle, hydroxymé- thyle, aminométhyle ou carboxyméthyle; un groupe représenté par la formule 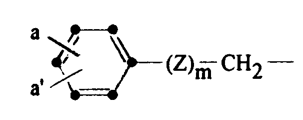
dans laquelle a et a' ont les mêmes significations que celles définies ci-dessus, Z représente O ou S, et m vaut 0 ou 1;
un groupe hétéroarylméthyle représenté par la formule
R₁-CH₂-
dans laquelle R₁ est un groupe thiényle, furyle, benzothiényle, benzofuryle, indolyle, triazolyle, tétrazolyle, oxazolyle, thiazolyle, oxadiazolyle, thiadiazolyle et de tels groupes hétéroaryles substitués par un groupe amino, hydroxyle, un atome d'halogène, un groupe alkyle en Ci à C₄, alcoxy en Ci à C₄, alkylsulfonylamino en Ci à C₄;
un groupe méthyle substitué représenté par la formule 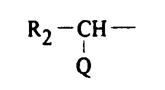
dans laquelle R₂ est un groupe cyclohex-1,4-diényle ou un groupe phényle facultativement substitué représenté par la formule 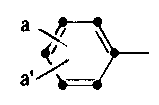
dans laquelle a et a' ont les significations définies ci-dessus, ou R₂ vaut R₁ tel que défini ci-dessus, et Q est un groupe hydroxyle, alcanoyloxy en Ci à C₄, carboxyle, sulfo, amino ou un groupe amino substitué représenté par la formule 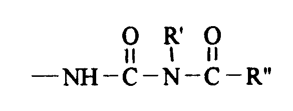
dans laquelle R" représente un groupe alkyle en Ci à C₄, furyle, thiényle, phényle, halophényle, nitrophényle, styryle, halostryryle, nitrostyryle ou un groupe 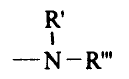
dans lequel chaque R' représente, indépendamment l'un de l'autre, un atome d'hydrogène ou un groupe alkyle en Ci à C₃, et R"' est un atome d'hydrogène, un groupe alkylsulfonyle en C₁ à C₃, alkyle en C₁ à C₃ ou alcanoyle en C₁ à C₄;
ou Q est un groupe amino substitué représenté par la formule 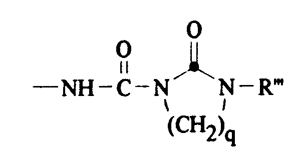
dans laquelle R"' a la même signification que celle définie ci-dessus et q vaut 2 ou 3;
ou Q est un groupe amino substitué représenté par la formule 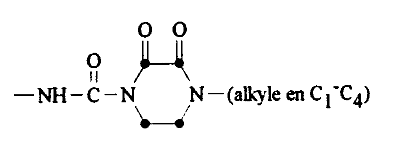 ou un groupe benzamido représenté par la formule 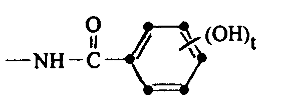
dans laquelle t vaut 1 à 3;
un groupe pyridone 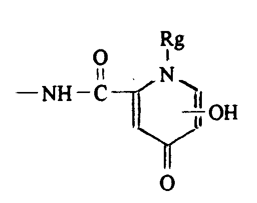 un groupe pyridyle 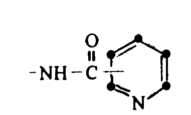
ou ledit groupe pytidyle substitué par un groupe alkyle en Ci à C₄, amino, carboxyle, hydroxyle ou un atome d'halogène; un groupe imidazolyle ou pyrazolyle 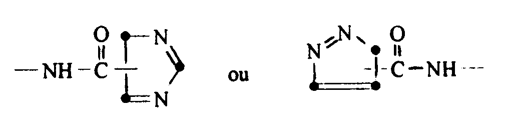
ou ledit groupe imidazolyle ou pyrazolyle substitué par un groupe alkyle en Ci à C₄, carboxyle, amino ou un atome d'halogène;
un groupe benzpyridazin-4-one ou un tautomère de celui-ci 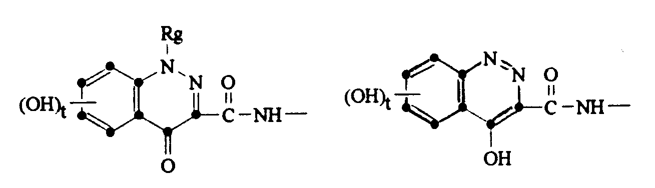
dans lequel Rg est un atome d'hydrogène ou un groupe alkyle en Ci à C₃, et t vaut 1 à 3;
ou un groupe benzpyranone 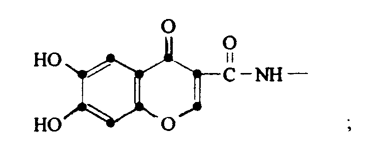 ou R est un groupe céto ou un groupe oximino-substitué représenté par les formules 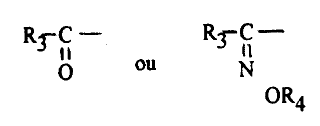
dans lesquelles R₃ vaut R₁ ou R₂ tels que définis ci-dessus et R₄ représente un atome d'hydrogène, un groupe alkyle en Ci à C₄ ou un groupe alkyle ou cycloalkyle carboxy-substitué représenté par la formule 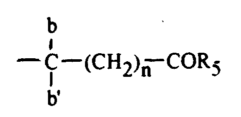
dans laquelle b et b' représentent, indépendamment l'un de l'autre, un atome d'hydrogène ou un alkyle en Ci à C₃, ou b et b', lorsqu'ils sont pris ensemble avec le carbone auquel ils sont attachés, forment un noyau carbocyclique de trois à six membres, n vaut 0 à 3 et R₅ est un groupe hydroxyle, alcoxy en Ci à C₄, amino, alkylamino en Ci à C₄ ou un groupe di(alkyle en Ci à C₄)amino;
Rₐ représente une charge négative, un atome d'hydrogène un groupe biologiquement instable ou un groupe carboxy-protecteur;
R₂ représente un atome d'hydrogène, un groupe alcoxy en Ci à C₄, alkylthio en Ci à C₄ ou un groupe formamido, -NHCHO;
- 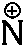 ≡Y représente un groupe ammonium quaternaire qui est
a. un noyau pyridinium, qui peut être substitué par un ou deux des substituants suivants : alkyle en Ci à C₆, alkyle en C₁ à C₆ substitué, cycloalkyle en C₃ à C₇, cycloalkyle en C₃ à C₇ substitué, cyclalcényle en C₅ à C₇, cyclalcényle en C₅ à C₇ substitué, halogène, cyano, hydroxyle, hydroxyle protégé, alcoxy en C₁ à C₄, alkylthio en C₁ à C₄, alkylsulfoxyde en C₁ à C₄, alkylsulfonyle en C₁ à C₄, phényle, phényle substitué, formyle, alcanoyle en C₂ à C₄, benzyle, benzoyle, amino, amino protégé, alkylamino en C₁ à C₄, di(alkyle en C₁ à C₄)amino, trifluorométhyle, carboxyle, carboxyle protégé, alcoxycarbonyle en C₁ à C₄, aminométhyle, aminométhyle protégé, carboxyméthyle, carboxyméthyle protégé, carbamoyle, qui peut être substitué une ou deux fois par un groupe alkyle en C₁ à C₆, un groupe aminosulfonyle (qui peut être substitué une ou deux fois par un groupe alkyle en C₁ à C₆), un acide sulfonique ou un groupe alkylène ou hétéroalkylène cyclique en C₂ à C₁₀ substitué ou non-substitué;
b. un noyau quinolinium, isoquinolinium, (1 ou 2)-pyradizinium, (1 ou 3)-pyrimidinium, N³⁻(alkyle en C₁ à C₄)imidazolinium, pyridazinium, phthalazinium, quinazolinium, purinium, pyrazinium, thiazolinium, isothiazolinium, oxazolinium, isoxazolinium, (3 ou 4)-1,3,4-thiadiazolinium, (2 ou 4)-1,2,4-thiadiazolinium, (2 ou 5)-1,2,5-thiadiazolinium, (3 ou 4)-1,3,4-oxadiazolinium, (2 ou 4)-1,2,4-oxadiazolinium ou (2 ou 5)-1,2,5-oxadiazolinium, ou leurs dérivés mono- ou disubstitués, dans lesquels les substituants peuvent être identiques ou différents (et dans le cas des noyaux quinolinium ou isoquinolinium, sur un ou sur les deux noyaux) et représentent un groupe amino, amino protégé, alkylamino en Ci à C₄, di(alkyle en Ci à C₄)amino, hydroxyle, hydroxyle protégé, alcoxy en Ci à C₄, alkylthio en Ci à C₄, alkylsulfoxyde en Ci à C₄, alkylsulfonyle en Ci à C₄, cyano, un atome d'halogène, un groupe alkyle en Ci à C₆, alkyle en Ci à C₆, substitué, trifluorométhyle. cycloalkyle en C₃ à C₇, cycloalkyle en C₃ à C₇ substitué, cyclalcényle en C₅ à C₇, cyclalcényle en C₅ à C₇ substitué, un acide sulfonique, un groupe carboxyle, carboxyle protégé, alcoxycarbonyle en Ci à C₄, hydroxyalkyle en Ci à C₃, hydroxyalkyle en Ci à C₃ protégé, formyle, alcanoyle en C₂ à C₄, un groupe aminosulfonyle (qui peut être substitué une ou deux fois par un groupe alkyle en Ci à C₆), carbamoyle (qui peut être substitué une ou deux fois par un groupe alkyle en C₁ à C₆), aminométhyle, aminométhyle protégé, carboxyméthyle, (carboxy protégé)méthyle, phényle, phényle substitué, benzoyle ou benzyle;
c. un groupe de formule 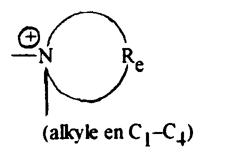 dans laquelle Rₑ, pris ensemble avec l'atome d'azote auquel il est lié, forment un noyau hétérocyclique de quatre à dix membres saturé ou partiellement insaturé, qui peut contenir un ou plusieurs hétéroatomes supplémentaires choisis parmi les atomes d'oxygène, d'azote ou de soufre et dans lequel le substituant peut être un groupe alkyle en C₁ à C₆, alkyle en C₁ à C₆ substitué, cycloalkyle en C₃ à C₇, cycloalkyle en C₃ à C₇ substitué, cyclalcényle en C₅ à C₇ cyclalcényle en C₅ à C₇ substitué, un atome d'halogène, un groupe cyano, hydroxyle, hydroxyle protégé, alcoxy en C₁ à C₄, alkylthio en C₁ à C₄, alkylsulfoxyde en C₁ à C₄, alkylsulfonyle en C₁ à C₄, phényle, phényle substitué, formyle, alcanoyle en C₂ à C₄, benzyle, benzoyle, amino, amino protégé, alkylamino en C₁ à C₄, di(alkyle en C₁ à C₄)amino, trifluorométhyle, carboxyle, carboxyle protégé, alcoxycarbonyle en C₁ à C₄, aminométhyle, aminométhyle protégé, carboxymêthyle, carboxyméthyle protégé, carbamoyle (qui peut être substitué une ou deux fois par un groupe alkyle en C₁ à C₆), aminosulfonyle (qui peut être substitué une ou deux fois par un groupe alkyle en C₁ à C₆) ou un acide sulfonique, ou les analogues benzo-fusionnés du noyau saturé ou partiellement insaturé, substitué ou non-substitué, ou
d. - 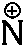 ≡Y représente un groupe ammonium tri(alkyle en C₁ à C₄); ou un solvate ou un sel non-toxique et pharmaceutiquement acceptable de ceux-ci, comprenant
a. la mise en réaction d'un ester 3-triflate de formule 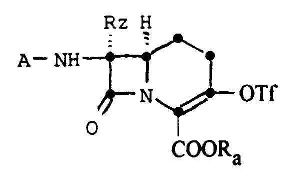 dans laquelle Tf est une fraction triflate (-S0₂CF₃) et A et Rₐ sont différents d'un atome d'hydrogène avec une amine N≡Y et, facultativement la déprotection du composé résultant; ou
b. l'estérification d'un composé de formule 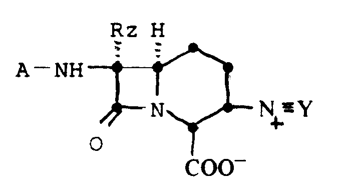 dans laquelle A est différent d'un atome d'hydrogène avec un groupe Rₐ⁺; ou c. la mise en réaction d'un composé de formule 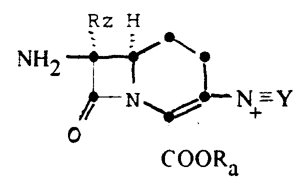 avec un réactif AX dans lequel A est different d'un atome d'hydrogene et X est un bon groupe sortant.

2. Procédé selon la revendication 1 dans lequel Rₐ est un atome d'hydrogène, une charge négative ou un groupe biologiquement instable et A est un groupe acyle RCO-.

3. Procédé selon la revendication 2 dans lequel R represente
R₂-CH(NH₂)- ou

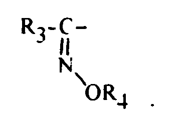

4. Procédé selon n'importe laquelle des revendications 2 ou 3 dans lequel -N=Y représente un noyau, substitué ou non-substitué, de pyridinium, quinolinium, isoquinolinium, 1- ou 2-pyridazinium, 1- ou 3- pyrimidinium, pyrazinium, thiazolinium, isothiazolinium, oxazolinium, isoxazolinium, 1,2,4-, 1,2,5- ou 1,3,4-thiadiazolinium ou -oxadiazolinium, ou un groupe de formule

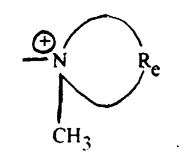

5. Procédé selon n'importe laquelle des revendications 2 à 4, dans lequel RCO-représente un groupe D-phénylglycyle, D-4-hydroxyphénylglycyle, D-2-thiénylglycyle, D-benzothièn-3-ylglycyle, (2-aminothiazol-4-yl)(méthoxyimino)acétyle ou (2-aminooxazol-4-yl)(méthoxyimino)acétyle.

6. Procédé pour la préparation d'une formulation pharmaceutique qui consiste dans le fait de mélanger un composé de Formule I tel que défini dans n'importe laquelle des revendications 2 à 5, ou un de ses solvates ou sels non-toxiques et pharmaceutiquement acceptables, avec un ou plusieurs supports, diluants ou excipients pharmaceutiquement acceptables pour celui-ci.
